# EUROPEAN PATENT SPECIFICATION

(11) **EP 1 459 075 B1**
(45) Date of publication and mention of the grant of the patent: **05.07.2006**
(21) Application number: 02796663.9
(22) Date of filing: 17.12.2002
(51) Int. Cl.: G01N 33/96, C12Q 1/68

(54) **NORMALISATION OF MICROARRAY DATA BASED ON HYBRIDISATION WITH AN INTERNAL REFERENCE**
NORMALISIERUNG VON MICROANORDNUNG-DATEN AUF DER BASIS VON HYBRIDISIERUNG MIT INTERNEM STANDARD
NORMALISATION DE DONNEES DE JEUX ORDONNES D'ECHANTILLONS BASEE SUR L'HYBRIDATION AVEC UNE REFERENCE INTERNE

(30) Priority: 21.12.2001 EP 01870295; 28.05.2002 US 383666 P
(43) Date of publication of application: 22.09.2004
(73) Proprietor: PamGene B.V., 5211 RX Den Bosch (NL)
(72) Inventor: VAN BEUNINGEN, Marinus, Gerardus, Johannes, NL-5345 RR Oss (NL)
(74) Representative: De Clercq, Ann G. Y.
(86) International application number: PCT/EP2002/014426
(87) International publication number: WO 2003/054551

(56) References cited:
- EP-A- 0 253 464
- WO-A-00/34523
- WO-A-00/65095
- WO-A-99/47706
- VAN GEMEN, B. ET AL: "A one tube quantitative HIV-1 RNA NASBA nucleic acid amplification assay using electrochemiluminescent (ECL) labelled probes" JOURNAL OF VIROLOGICAL METHODS, vol. 49, no. 2, 1994, pages 157-168, XP000600186
- PATEL, RAJESH ET AL: "Quantification of HIV-1 RNA using a homogeneous single-tube assay" CLINICAL CHEMISTRY, vol. 46, no. 9, 2000, pages 1487-1488, XP001071218
- VAN BEUNINGEN, R ET AL: "Development of a high throughput detection system for HIV-1 using real-time NASBA based on molecular beacons" PROCEEDINGS OF SPIE, vol. 4264, 2001, pages 66-71, XP001078979
- VAN BEUNINGEN, R. ET AL: "Fast and specific hybridization using flow-through microarrays on porous metal oxide" CLINICAL CHEMISTRY, vol. 47, no. 10, 12 December 2001 (2001-12-12), pages 1931-1933, XP002200111

## Description

### FIELD OF THE INVENTION

The invention relates to methods and corresponding arrays especially suited to correct for signal errors due to variations in sample preparation. Methods and compositions for performing quantitative array-based assays are provided. In the subject methods, both a reporter and an analyte is employed, where the reporter is characterized by binding selectively to an internal reference present on the array, i.e. at least a subset of, if not all of, the spots present on the array employed in the method contain an internal reference which can be bound by the reporter.

### BACKGROUND OF THE INVENTION

Microarrays of binding agents, such as oligonucleotides and peptides, have become an increasingly important tool in the biotechnology industry and related fields. These binding agent arrays, in which a plurality of binding agents are deposited onto a substrate, often a solid substrate, in the form of an array or pattern, find use in a variety of applications, including drug screening, nucleic acid sequencing, mutation analysis, genotyping, expression profiling, genetic abnormality screening by MAPH and the like. One important use of microarrays is in the analysis of differential gene expression, where the expression of genes in different cells, normally a cell of interest and a control, is compared and any discrepancies in expression are identified. In such assays, the presence of discrepancies indicates a difference in the classes of genes expressed in the cells being compared.

In methods of differential gene expression, arrays find use by serving as a substrate to which "probe" fragments or "receptors", such as for example polynucleotides, are bound. One then obtains "targets" or "analytes" from analogous cells, tissues or organs of, e.g. a healthy and diseased organism. The targets are next hybridized to the immobilized set of polynucleotide "probe" fragments. Differences between the resultant hybridization patterns are subsequently detected and related to differences in gene expression in the two sources.

Because of the varied and important information that microarrays can provide, as well as the many potential applications of such devices, the use of these microarrays in research, diagnostic and related applications has grown considerably and is expected to continue to do so. A variety of different array technologies have been developed in order to meet the growing need of the biotechnology industry, as evidenced by the extensive number of patents and other literature published.

However, there are disadvantages with current protocols. For example, the efficiency of hybridization of target nucleic acids to the array can be limited by experimental limitations, e.g. differences in sample preparation or different target nucleic acids can have different hybridization efficiencies to the probe nucleic acids of the array. Differences in hybridization efficiency result in differences in the intensity of hybridization to different probe nucleic acids of the array, even though the targets are present in equivalent concentrations. Where two or more arrays are employed in a particular application, e.g. in gene expression analysis, variation in the quality of array (reproducibility of array production), and in assay conditions between the different arrays can preclude direct comparison of data obtained on the arrays, since conditions such as hybridization time, probe labeling, and detection procedures may differ, and variations between the arrays may be present. All of these errors result in spot to spot variation. Furthermore, it is difficult to compare data generated by using different types of oligonucleotide or polynucleotide based arrays. Concentration of target nucleic acids in a sample cannot be compared between arrays produced by different methods and/or manufacturers based on intensity of signals because the set of probe sequences often differs between arrays. Thus, the signal error obtained in arrays is the sum of all the individual errors such as the inhomogeneous substrate activation, liquid dispense volume variation, probe coupling differences, temperature variation, flow variation, optical aberrations, et cetera. As a result, current array technology is used mainly for discovery of differentially-expressed genes rather than for any specific quantitative assay. In this respect, two formats are generally employed: (a) comparison of two hybridization patterns to each other and (b) simultaneous hybridization to the same array of two different targets derived from two different biological sources and labeled by different labels. In the latter approach, which is more commonly employed, fold differences in gene expression between the two samples are often measured.

In these application areas, as well as others, it is important to significantly distinguish between different mRNA expression levels and genetic copy number differences as small as 1.5 fold. This requires that all aspects of the system should be discriminative over a signal difference of only 1.5 fold.

As such, there is a continued need for the development of additional arrays and array-based protocols.

Of interest would be the development of an array-based methodology that incorporates an internal calibration standard, where such a method would eliminate variations resulting from the quality of the array, the type of the array, the quality of the assay conditions, and the like. In addition, there is a need for an array-based protocol that provides quantitative data about sample preparation, target concentration, and a corresponding method of quantification to allow more accurate comparison of data between arrays.

WO 00/34523 by Hyseq Inc describes the addition of a detectable label which is proportional to the amount immobilized at a certain spot, to correct for probe coupling differences during the preparation of the assays. Similarly, WO 00/65095 by Clontech Laboratories Inc relates to the normalisation for differences in immobilization efficiencies of the probes at different addresses in the array.

Evidently, the prior art relates only to the manufacture of arrays and spot to spot variation, but not to signal errors due to analyte processing. Meanwhile, contemporary microarrays are produced by established techniques, resulting generally in qualitatively highly reliable products. The predominant quality problem resides in the sample preparation.

### SUMMARY OF THE INVENTION

In order to measure individual differences and subsequently correct for these differences the present invention provides microarrays comprising a substrate with predefined regions, wherein each binding substance immobilized at a predefined region of said substrate comprises a receptor and a predetermined amount of an internal reference, wherein the signal generated by said internal reference is determined by means of a reporter molecule and wherein said reporter molecule selectively binds to said internal reference. Moreover, the present invention provides a method for the identification of an analyte in a sample, such as for example a biological sample, comprising the steps of :
(a) providing a microarray comprising a substrate wherein each binding substance immobilized onto said substrate comprises a predetermined amount of receptor and a predetermined amount of an internal reference,
(b) providing a reporter molecule that binds selectively to said internal reference,
(c) adding said reporter molecule to said sample,
(d) contacting said sample comprising said reporter molecule with said microarray under conditions that allow binding to take place between said receptor and said analyte, and between said internal reference and said reporter molecule,
(e) determining the signal of said reporter molecule binding to said internal reference,
(f) determining the signal of said analyte binding to said receptor, and,
(g) normalising said signal of step (f) for said signal of step (e).

### DETAILED DESCRIPTION

The invention relates to methods and corresponding microarrays especially suited to correct for signal errors due to variations in sample preparation. Methods and compositions for performing quantitative microarray-based assays are provided. In the subject methods, both a reporter and an analyte is employed, where the reporter is characterized by binding selectively to an internal reference present on the array, i. e. at least a subset of, if not all of, the spots present on the array employed in the method contain an internal reference which can be bound by the reporter.

In this specification and the appended claims, the singular forms "a," "an," and "the" include plural references unless the context clearly dictates otherwise. Unless defined otherwise, all technical and scientific terms used herein have the same meaning as commonly understood to one of ordinary skill in the art to which this invention belongs.

Generally, in order to analyse a sample on a microarray, the sample is manipulated before it is contacted to said microarray. Manipulations include, for example, cDNA production from RNA, production and/or isolation of nucleic acids, antibodies, polypeptides and the like. Each step of this manipulation of a sample can introduce errors, such as in amount or integrity of the molecule of interest. This problem is extremely manifest if two or more samples need to be compared. The present invention relates to the normalisation of signals of samples containing an analyte via adding a predetermined amount of a reporter to this sample. The internal reference is eventually used for normalising sample to sample variation due to the processing of the samples (between samples normalisation), as well as variations observed with one subject sample, such as for example due to spot to spot variation in a given microarray (within sample normalisation).

In particular, the present invention provides a method for the identification of an analyte in a sample comprising the steps of :
(a) providing a microarray comprising a substrate wherein each binding substance immobilized onto said substrate comprises a predetermined amount of receptor and a predetermined amount of an internal reference,
(b) providing a reporter molecule that binds selectively to said internal reference,
(c) adding said reporter molecule to said sample,
(d) contacting said sample comprising said reporter molecule with said microarray under conditions that allow binding to take place between said receptor and said analyte, and between said internal reference and said reporter molecule,
(e) determining the signal of said reporter molecule binding to said internal reference,
(f) determining the signal of said analyte binding to said receptor, and,
(g) normalising said signal of step (f) for said signal of step (e).

The term "analyte in a sample" refers to a molecule in a sample, i.e. a molecule to be analysed which is present in a sample. The molecules in a sample can be, e.g. nucleic acids (both DNA and RNA), peptides, polypeptides, proteins, antibodies, carbohydrates, and/or small biomolecules (e.g. drug candidates). The sample can be, for example, a physiological or a biological sample.

Samples are generally manipulated in order to isolate and/or characterise the analyte. For example, analyte nucleic acids are generally isolated from a biological sample (cells, tissues, organs, etc.), processed and converted to other nucleic acids using known in the art technology, such as PCR, reverse transcription, etc., e.g. mRNA, cDNA, PCR products, cRNA, and the like. The analyte nucleic acids may be isolated from a tissue or cell of interest using any method known in the art. Total RNA or its transcriptionally active fraction mRNA can be isolated from a tissue and labeled and used directly as analyte nucleic acid, or it may be converted to a labeled cDNA, cRNA, etc. via methods such as reverse transcription, transcription, Tyras, NASBA and/or PCR. Generally, such methods will employ the use of oligonucleotide primers, and the primers can be anchored by bacteriophage RNA polymerase promoter. The primers may be designed to copy a large spectrum of RNA species, e.g. oligo (dT) primers or random hexamers, or designed specifically to copy a subset of genes of interest. After the copying step, i.e. conversion of mRNA to cDNA, cDNA can be amplified by PCR or by linear amplification using bacteriophage RNA polymerase mediated transcription, NASBA or Tyras. As with the reporter nucleic acids, in a preferred embodiment the analyte nucleic acid sequences are generated using a set of a representative number of gene specific primers.

In the present invention, the term "reporter" refers to a molecule that corresponds, e.g. interacts with or binds to, an internal reference, which is covalently bound to the substrate of the array. The reporter is added to a sample, before said sample is contacted to an array. The reporter can be added at different steps of sample manipulation. It will be clear that if a reporter is added in the first step(s) of sample manipulation, then the reporter will undergo the same or most of the steps of the manipulation which the sample undergoes.

For example, reporter and analyte nucleic acids may be hybridized to the array and/or detected simultaneously. Thus, reporter and analyte nucleic acids may be combined prior to hybridization and the array hybridized to both simultaneously to minimize potential variability in hybridization conditions. For example, a known amount of labeled reporter and the analyte nucleic acids can be added to the same hybridization buffer, and then contacted with one or more arrays simultaneously under hybridization conditions. In another example, a known amount of labeled reporter and analyte nucleic acids are added to the same hybridization mix, and this buffer aliquoted for the separate hybridization of different arrays. By storing aliquots of the hybridization mix (e. g. storage at -20 °C or -70 °C), different arrays may be hybridized at different times with approximately the same amounts of the mix.

The term "target" refers to a sample to be analysed. Said target may comprise the analyte and/or the reporter.

Another feature of reporters added to the analyte in a sample is that the concentration and/or amount of the reporter is known.

At the moment of adding the reporter to the sample, the reporter should be structurally as similar as possible to the analyte in the sample. Hence, the reporter can be, e.g. nucleic acids (both DNA and RNA), peptides, polypeptides, proteins, antibodies, carbohydrates, and/or small biomolecules. For example, when the analyte is RNA that is converted to cDNA, then the reporter should preferably be also RNA. In other words, the structure of the reporter molecule should be as similar as possible to that of the analyte in order to maximally imitate the binding, e.g. hybridization, of the analyte to, e.g. target nucleic acid.

Reporter nucleic acids may be the same length, shorter or longer than their corresponding internal reference sequences on the array or analyte nucleic acid in the sample (if present).

However, each reporter nucleic acid should have a least partial complementarity to its corresponding internal reference nucleic acid. In addition, the reporter nucleic acid should have structural and hybridization characteristics very similar to its corresponding analyte nucleic acid, e.g. it should have similar hybridization efficiencies, similar kinetics with complementary probe sequences, similar background hybridization with other sequences, etc. For example, where the analyte set of nucleic acids comprises labeled cDNAs reverse transcribed from a control set of a representative pool of synthetic RNAs, the reporter nucleic acids will also generally be labeled cDNAs reverse transcribed from mRNAs, e.g. synthetic mRNAs.

Each internal reference nucleic acid may be the same length as its corresponding reporter nucleic acid, longer than its corresponding reporter nucleic acid or shorter than its corresponding reporter nucleic acid. In general, the length of each reporter nucleic acid or set of reporter nucleic acids in a given sample is at least about 25 nucleotides, or at least about 50 nucleotides; or at least about 100 nucleotides, where the length could be as a long as 2 kb or longer, but will generally not exceed about 1 kb and more usually will not exceed about 800 nucleotides.

The reporter nucleic acid may be synthetic nucleic acids or isolated from a biological source. The reporter nucleic acids may be generated using any convenient protocol, including reverse transcription protocols (e.g. using AMV or MoMLV reverse transcriptase), bacteriophage RNA polymerase (T7 RNA polymerase, T3 RNA polymerase, etc.) mediated transcription, PCR-, NASBA- or Tyras-protocols, oligonucleotide synthesis protocols (e.g. nucleotide chemistry), and the like. In an embodiment, the reporter nucleic acid sequences are generated using cDNA fragments cloned into appropriate expression vectors using a set of a representative number of gene specific primers. These cloned cDNAs are then used to produce RNA control targets using techniques such as PCR and/or bacteriophage RNA polymerase mediated transcription, NASBA or Tyras. Of interest are applications in which the gene specific primers used to generate the reporter are the same as the gene specific primers used to generate the analyte nucleic acids is employed.

After synthesis, each reporter nucleic acid is quantitated using procedures such as spectrophotometry, fluorescence measurement, etc. Known quantitative amounts of each reporter nucleic acid are mixed with the sample for sample preparation or, directly, for use in hybridization assays, as described herein. In another embodiment, the reporter nucleic acids are mixed together in equal molar amounts, at predetermined ratios, at equal weight amounts, etc, where in many embodiments they will be mixed together in equal weight amounts, such that the amount of each individual reporter nucleic acid in the sample is the same as the analyte nucleic acid in the sample.

Each reporter molecule should bind to its corresponding internal reference with selectivity and sensitivity. A reporter nucleic acid that selectively binds with its corresponding internal reference nucleic acid is for example at least 10 times, at least 100 times, or at least 1000 times more likely to bind with its designated internal reference nucleic acid than to a non-specific nucleic acid, and preferably any other sequence present on the array. Non-specific nucleic acids include those of random sequence, coding sequences found in a particular array other than the designated internal reference nucleic acid, and coding sequences of non-internal reference sequences specific to the organism from which the internal reference nucleic acids are derived.

Reporter nucleic acids of the invention also display sufficient sensitivity upon binding with their designated internal reference nucleic acids. By "sufficient sensitivity" is meant that binding of the reporter nucleic acid is significantly greater than the binding of background nucleic acids of random sequence, where the strength of binding is for example at least 10 times, at least 100 times, or at least 500 times greater than the recognition of background nucleic acids of random sequence. In many embodiments, the nucleotide sequences of the subject reporter nucleic acids are chosen with algorithms, where such algorithms are described in detail in PCT publication WO 97/10365 and PCT/US96/14839.

A wide variety of different molecules can be immobilized on the substrate of the present arrays. Similarly, the present methods are applicable to a wide variety of different molecules or receptors that may be placed on the substrate of the arrays. The methods and arrays are particularly exemplified herein in terms of polynucleotides immobilized on a substrate, but they are equally applicable to other types of molecules. For example, one of skilled in the art could easily adapt the present methods and arrays to apply to other nucleic acids (both DNA and RNA), peptides, polypeptides, proteins, antibodies, carbohydrates, small biomolecules (e.g. drug candidates), or any other types of molecule that can be immobilized on a substrate by any method.

The terms "predefined region" or "spot" are used interchangeably throughout the present invention. The latter terms relate to individually, spatially addressable positions on an array.

In the present invention, a binding substance is immobilized on the substrate at a spatially predefined region, i.e. at a particular spot. The binding substance comprises at least a receptor and a predetermined amount of an internal reference. The binding substance does not refer to or preclude a linking between the receptor and the internal reference.

### For example, the receptor and the internal reference are separate molecules

In this regard, the term "receptor" refers to any molecule stably associated with a substrate which corresponds to a target molecule of interest or analyte in a sample, if present. Receptors are not random molecules, but are predefined.

The term "internal reference" refers to any molecule stably associated with a substrate which corresponds to a reporter molecule. The reporter molecule is designed to specifically bind or attach to the internal reference.

The internal references are structurally as similar as possible to the receptors that are employed in the assays, e.g. both sets of internal references and receptors are nucleic acids. In other words, the structure of the internal reference should be similar to that of the receptor in order to maximally imitate the binding, e.g. hybridization.

Accordingly, the present invention relates to methods as described herein, wherein said internal reference comprises nucleic acids, polynucleic acids or (poly)peptides or chemical compounds.

Accordingly, the present invention relates to methods as described herein, wherein said reporter molecule comprises polynucleic acids or (poly)peptides or chemical compounds.

A critical feature of the arrays of the invention is the predetermined amounts of the receptors and the internal reference. It will be appreciated by the man skilled in the art that the receptor and the internal reference may be a hybrid, i.e. the receptor and the internal reference are covalently bound to each other, or the receptor and the internal reference reside on the same molecule, e.g. a fusion protein. For example, a nucleic acid containing two regions, e.g. a hybrid, of which one region, i.e. internal reference, corresponds to the reporter, while another region, i.e. receptor, corresponds to the analyte. In case of a hybrid, the amount of the internal reference correlates directly to the amount of the receptor.

Accordingly, the present invention relates to a method as described herein, wherein each binding substance immobilized onto said substrate comprises at least 1 % to at most 99% of said internal reference.

Accordingly, the present invention relates to a method as described herein, said each binding substance immobilized onto said substrate comprises the same predetermined amount of said internal reference.

Non-receptor sequences, e.g. control nucleic acids, on the array may not have a target or corresponding nucleic acid in the analyte or reporter set, e.g. array sequences such as orientation sequences, negative and positive control sequences, etc. that may be present on an array.

The term "nucleic acid" as used herein means a polymer composed of nucleotides, e.g. deoxyribonucleotides or ribonucleotides. The terms "ribonucleic acid" and "RNA" as used herein means a polymer composed of ribonucleotides. The terms "deoxyribonucleic acid" and "DNA" as used herein means a polymer composed of deoxyribonucleotides. The term "oligonucleotide" as used herein denotes single stranded nucleotide multimers of from about 10 to about 100 nucleotides in length. The term "polynucleotide" as used herein refers to single or double stranded polymer composed of nucleotide monomers of from about 10 to about 100 nucleotides in length, usualy of greater than about 100 nucleotides in length up to about 1000 nucleotides in length.

The microarrays of the present invention may be of any desired size, from two spots to 10⁶ spots or even more. The upper and lower limits on the size of the substrate are determined solely by the practical considerations of working with extremely small or large substrates.

For a given substrate size, the upper limit is determined only by the ability to create and detect the spots in the microarray. The preferred number of spots on a microarray generally depends on the particular use to which the microarray is to be put. For example, sequencing by hybridization will generally require large arrays, while mutation detection may require only a small array. In general, microarrays contain from 2 to about 10⁶ spots, or from about 4 to about 10⁵ spots, or from about 8 to about 10⁴ spots, or between about 10 and about 2000 spots, or from about 20 to about 200 spots.

Furthermore, not all spots on the microarray need to be unique. Indeed, in many applications, redundancies in the spots are desirable for the purposes of acting as internal controls.

A variety of techniques have been described for synthesizing and/or immobilizing arrays of polynucleotides, including *in situ* synthesis, where the polynucleotides are synthesized directly on the surface of the substrate (see, e.g., U.S. Pat. No. 5,744,305 to Fodor, *et al*.,) and attachment of pre-synthesized polynucleotides to the surface of a substrate at discrete locations (see, e.g., WO 98/31836). Additional methods are described in WO 98/31836 at pages 41-45 and 47-48, among other places. The present invention is suitable for use with any of these currently available, or later developed, techniques.

Immobilization of pre-synthesized polynucleotides at different spatial addresses yields an array of polynucleotides whose sequences are identifiable by their spatial addresses.

In embodiments involving *in situ* synthesis of polynucleotides, the polynucleotides are synthesized in their usual manner. The synthetic scheme yields an array of polynucleotides whose sequences are identifiable by their spatial addresses.

While the above method contemplates labeling the last nucleotide of the polynucleotide, those of skill in the art will appreciate that other positions, or additional positions, could be similarly labeled to provide information about the proportions of truncated polynucleotides synthesized. In these embodiments, the labels used at the various steps should be distinguishable from one another.

Moreover, while the *in situ* synthesis method is described utilizing phosphoramidite reagents, it will be recognized that other reagents utilizing other synthesis strategies can also be employed, and in certain circumstances may be preferable, depending on the stability of the chosen label to the synthesis conditions. Non-limiting examples of suitable chemistries and reagents are described, for example in Oligonucleotide Synthesis: A Practical Approach, M. J. Gait, Ed., IRL Press, Oxford, England, 1985.

The composition of the immobilized polynucleotides, e.g. receptors and internal references, is not critical. The only requirement is that they be capable of hybridizing to a target nucleic acid of complementary sequence, e.g. reporters and analytes, if any. For example, the polynucleotides may be composed of all natural or all synthetic nucleotide bases, or a combination of both. Non-limiting examples of modified bases suitable for use with the instant invention are described, for example, in Practical Handbook of Biochemistry and Molecular Biology, G. Fasman, Ed., CRC Press, 1989, pp. 385-392. While in most instances the polynucleotides will be composed entirely of the natural bases (A, C, G, T or U), in certain circumstances the use of synthetic bases may be preferred.

Moreover, while the backbones of the polynucleotides will typically be composed entirely of "native" phosphodiester linkages, they may contain one or more modified linkages, such as one or more phosphorothioate, phosphoramidite or other modified linkages. As a specific example, one or more immobilized polynucleotides may be a peptide nucleic acid (PNA), which contains amide interlinkages. Additional examples of modified bases and backbones that can be used in conjunction with the invention, as well as methods for their synthesis can be found, for example, in Uhlman & Peyman, 1990, Chemical Review 90(4):544-584; Goodchild, 1990, Bioconjugate Chem. 1(3):165-186; Egholm *et al.,* 1992, J. Am. Chem. Soc. 114:1895-1897; Gryaznov *et al.,* J. Am. Chem. Soc. 116:3143-3144, as well as the references cited in all of the above.

As such, the internal reference and receptor nucleic acids may include polymers of ribonucleotides and deoxyribonucleotides, with the ribonucleotide and/or deoxyribonucleotides being connected together via 5' to 3' linkages. Internal reference and receptor nucleic acids of the invention may be ribonucleic acids, for example sense or antisense ribonucleic acids, full-length or partial fragments of cRNA, full-length or partial fragments of mRNA, and/or ribo-oligonucleotides. Alternatively, internal reference and receptor nucleic acids of the invention may be deoxyribonucleic acids, preferably single-stranded full-length or fragments of sequences encoding the corresponding mRNAs. The form of the internal reference and receptor nucleic acids should be chosen so that they are complimentary to and form appropriate Watson-Crick hydrogen bonds with reporter and analyte present in a sample. For example if analyte sequences in a sample correspond in sequence to mRNA, then internal reference and receptor sequences should be complementary, e. g. antisense or complementary RNA (cRNA).

As mentioned above, the internal reference and receptor nucleic acids may be polymers of synthetic nucleotide analogs. Such internal reference and receptor nucleic acids may be utilised in certain embodiments because of their superior stability under assay conditions. Modifications in the native structure, including alterations in the backbone, sugars or heterocyclic bases, have been shown to increase intracellular stability and binding affinity. Among useful changes in the backbone chemistry are phosphorothioates; phosphorodithioates, where both of the non-bridging oxygens are substituted with sulfur; phosphoroamidites; alkyl phosphotriesters and boranophosphates. A-chiral phosphate derivatives include 3'-O-5'-S-phosphorothioate, 3'-S-5'-O-phosphorothioate, 3'-CH₂-5'-O-phosphonate and 3'-NH-5'-O-phosphoroamidate. Peptide nucleic acids replace the entire ribose phosphodiester backbone with a peptide linkage. Locked nucleic acids give additional conformational stability of sugar moiety due to additional bonds between 2'-carboxyl and 5' carboxyl or 4'-carboxyl groups of deoxyribose. Sugar modifications are also used to enhance stability and affinity. The a-anomer of deoxyribose may be used, where the base is inverted with respect to the natural p-anomer. The 2'-OH of the ribose sugar may be altered to form 2'-O-methyl or 2'-O-allyl sugars, which provides resistance to degradation without comprising affinity. Modification of the heterocyclic bases that find use in the method of the invention are those capable of appropriate base pairing. Some useful substitutions include deoxyuridine for deoxythymidine; 5-methyl-2'-deoxycytidine and 5-bromo-2'-deoxycytidine for deoxycytidine. 5-propynyl-2'-deoxyuridine and 5-propynyl-2'-deoxycytidine have been shown to increase affinity and biological activity when substituted for deoxythymidine and deoxycytidine, respectively.

Examples of non-naturally occurring bases that are capable of forming base-pairing relationships include, but are not limited to, aza- and deaza-pyrimidine analogues, aza- and deaza-purine analogues, and other heterocyclic base analogues, wherein one or more of the carbon and nitrogen atoms of the purine and pyrimidine rings have been substituted by heteroatoms, e.g., oxygen, sulfur, selenium, phosphorus, and the like.

The immobilized polynucleotides may be as few as four, or as many as hundreds, or even more, nucleotides in length. Contemplated as polynucleotides according to the invention are nucleic acids that are typically referred to in the art as oligonucleotides and also those referred to as nucleic acids. Thus, the arrays of the present invention are useful not only in applications where target nucleic acids are hybridized to immobilized arrays of relatively short (such as, for example, having a length of approximately 6, 8, 10, 20, 40, 60, 80, or 100 nucleotides) probes, but also in applications where relatively short probes are hybridized to arrays of immobilized nucleic acids.

The polynucleotides of the array can be of any desired sequence. In a preferred embodiment, they can comprise all possible polynucleotides of a given length N, which would result in an array of 4^{N} unique elements. For all polynucleotides of, for example, 6 bases in length, the sequences would comprise an array with 4096 unique elements.

Alternatively, the polynucleotides can make up the "universal set" for sequencing a nucleic acid, as discussed in WO 98/31836, particularly pages 27-29.

In an alternative embodiment, the set of polynucleotides may correspond to particular mutations that are to be identified in a known sequence. For example, if a particular nucleic acid is known to contain an unidentified mutation at a particular position, then the mutated position can be identified with an array of eight polynucleotides, three corresponding to the three possible substitutions at that position, one corresponding to the deletion of the base at that position, and four corresponding to the insertion of the four possible bases at that position. Alternatively, for a known gene that may contain any of several possible identified mutations, the array can comprise polynucleotides corresponding to the different possible mutations. This embodiment is, for instance, useful for genes like oncogenes and tumor suppressors, which frequently have a variety of known mutations in different positions. Using arrays facilitates determining whether or not these genes contain mutations by allowing simultaneous screening with polynucleotides corresponding to each of these different positions.

In another alternative embodiment, each spot of the array can comprise a mixture of polynucleotides of different sequences. These mixtures may comprise degenerate polynucleotides of the structure Nx By Nz, wherein N represents any of the four bases and varies for the polynucleotides in a given mixtures, B represents any of the four bases but is the same for each of the polynucleotides in a given mixture, and x, y, and z are integers.

Arrays comprising this type of mixture are useful in, for example, sequencing by hybridization. Alternatively, the spots may comprise mixtures of polynucleotides that correspond to different regions of a known nucleic acid; these regions may be overlapping, adjacent, or nonadjacent. Arrays comprising these types of mixtures are useful in, for example, identifying specific nucleic acids, including those from particular pathogens or other organisms. Both types of mixtures are discussed in WO 98/31836, particularly at pages 123-128.

The polynucleotides intended for receptors can be isolated from biological samples, generated by PCR-, NASBA-, Tyras-reactions or other template-specific reactions, or made synthetically. Methods for isolating polynucleotides from biological samples and/or PCR-, Tyras-, NASBA-reactions are well-known in the art, as are methods for synthesizing and purifying synthetic polynucleotides. Probes isolated from biological samples and/or PCR- Tyras-, NASBA-reactions may, depending on the desired mode of immobilization, require modification at the 3'- or 5'-terminus, or at one or more bases, as will be discussed more thoroughly below. Moreover, since the polynucleotide must be capable of hybridizing to a target nucleic acid, if not already single stranded, it should preferably be rendered single stranded, either before or after immobilization on the substrate.

The polynucleotides can be immobilized on the substrate using a wide variety of techniques. For example, the polynucleotides can be adsorbed or otherwise non-covalently associated with the substrate (for example, immobilization to nylon or nitrocellulose filters using standard techniques); they may be covalently attached to the substrate; or their association may be mediated by specific binding pairs, such as biotin and streptavidin.

In order to effect covalent attachment, the substrate must first be activated, i.e., treated so as to create reactive groups on or within the substrate that can react with a reactive group on the polynucleotide to form a covalent linkage. Those of skill in the art will recognize that the desired reactive group will depend on the chemistry used to attach the polynucleotides to the substrate and the composition of the substrate. Typical reactive groups useful for effecting covalent attachment of polynucleotides to substrates include hydroxyl, aldehyde, sulfonyl, amino, epoxy, isothiocyanate and carboxyl groups; however, other reactive groups as will be apparent to those having skill may also be used and are also included within the scope of the invention.

For a review of the myriad techniques that can be used to activate the substrates with suitable reactive groups, see Wiley Encyclopedia of Packaging Technology, 2d Ed., Brody & Marsh, Ed., "Surface Treatment," pp. 867-874, John Wiley & Sons (1997), and the references cited therein (hereinafter "Surface Treatment"). Chemical methods suitable for generating amino groups on silicon oxide substrates are described in Atkinson & Smith, "Solid Phase Synthesis of Oligodeoxyribonucleotides by the Phosphite Triester Method," In: Oligonucleotide Synthesis: A Practical Approach, M J Gait, Ed., 1984, IRL Press, Oxford, particularly at pp. 45-49 (and the references cited therein); chemical methods suitable for generating hydroxyl groups on silicon oxide substrates are described in Pease *et al.,* 1994, Proc. Natl. Acad. Sci. USA 91:5022-5026 (and the references cited therein); chemical methods for generating functional groups on polymers such as polystyrene, polyamides and grafted polystyrenes are described in Lloyd-Williams *et al.,* 1997, Chemical Approaches to the Synthesis of Peptides and Proteins, Chapter 2, CRC Press, Boca Raton, Fla. (and the references cited therein).

It is contemplated that in general the binding substance is covalently bound to the substrate. This minimises loss of the binding substance from the substrate. Covalent binding of an organic compound to a metal oxide is well known in the art, for example using the method described by Chu. C.W., *et al.* (J. Adhesion Sci. Technol., 7, pp.417-433, 1993) and Fadda, M.B. *et al.* (Biotechnology and Applied Biochemistry, 16, pp. 221-227, 1992). Further, after activation of a metal oxide support by a silanating agent and binding of the biomolecules, a number of amino-groups of said silanating agent can still be present as unloaded amino-groups. This may result in unwanted interactions of said amino-groups with various substances present in the medium in which the loaded support is used, resulting in high background signals. The unloaded amino-groups can be removed from the support without affecting the loaded part of the support by subsequently treating the loaded support with an acidic solution. Similarly, an activated and loaded support may be treated with a basic or neutral solution, provided that the method is not used for derivatization of aluminiumoxide nanoparticles aminated with (3-aminopropyl)-triethoxysilane, wherein the basic solution further contains a large excess of N-acetylhomocysteinelactone. In this regard, the European patent application PCT/EP00/07736 is exemplary.

Those of skill in the art will recognize that in embodiments employing covalent attachment, the covalent bond formed between the polynucleotide and the substrate must be substantially stable to the various conditions under which the array will be assayed, to avoid loss of polynucleotide during the assay. One such stable bond is the phosphodiester bond, which connects the various nucleotides in a polynucleotide, and which can be conveniently formed using well-known chemistries (see, e.g., Oligonucleotide Synthesis: A Practical Approach, 1984, supra). Other stable bonds suitable for use with hydroxyl-activated substrates include phosphorothioate, phosphoramidite, or other modified nucleic acid interlinkages. For substrates modified with amino groups, the bond could be a phosphoramidate, amide or peptide bond. When substrates are activated with epoxy functional groups, a stable C-N bond could be formed. Suitable reagents and conditions for forming such stable bonds are well known in the art. Other stable bonds suitable for use with the arrays of the invention will be apparent to those of skill in the art.

In embodiments in which pre-synthesized polynucleotides are covalently attached to the substrate, the polynucleotides may be attached via their 3'-terminus, 5'-terminus or by way of a reactive group at one of the bases. Synthesis supports and synthesis reagents useful for modifying the 3'- and/or 5'-terminus of synthetic polynucleotides, or for incorporating a base modified with a reactive group into a synthetic polynucleotide, are well-known in the art and are also commercially available.

For example, methods for synthesizing 5'-modified polynucleotides are described in Agarwal *et al.,* 1986, Nucl. Acids Res. 14:6227-6245 and Connelly, 1987, Nucl. Acids Res. 15:3131-3139. Commercially available products for synthesizing 5'-amino modified polynucleotides include the N-TFA-C6-AminoModifier, N-MMT-C6-AminoModifier and N-MMT-C12-AminoModifier reagents available from Clontech Laboratories, Inc., Palo Alto, California.

Methods for synthesizing 3 '-modified polynucleotides are described in Nelson *et al.,* 1989, Nucl. Acids Res. 17:7179-7186 and Nelson *et al.,* 1989, Nucl. Acids Res. 17:7187-7194. Commercial products for synthesizing 3'-modified polynucleotides include the 3'-Amino-ON.TM. controlled pore glass and Amino Modifier II.TM. reagents available from Clontech Laboratories, Inc., Palo Alto, Calif.

Other methods for modifying the 3' and/or 5' termini of polynucleotides, as well as for synthesizing polynucleotides containing appropriately modified bases are provided in Goodchild, 1990, Bioconjugate Chem. 1:165-186, and the references cited therein. Chemistries for attaching such modified polynucleotides to substrates activated with appropriate reactive groups are well-known in the art (see, e.g., Ghosh & Musso, 1987, Nucl. Acids Res. 15:5353-5372; Lund *et al.,* 1988, Nucl. Acids Res. 16:10861-10880; Rasmussen *et al.,* 1991, Anal. Chem. 198:138-142; Kato & Ikada, 1996, Biotechnology and Bioengineering 51:581-590; Timofeev *et al.,* 1996, Nucl. Acids Res. 24:3142-3148; O'Donnell *et al.,* 1997, Anal. Chem. 69:2438-2443).

Methods and reagents for modifying the ends of polynucleotides isolated from biological samples and/or for incorporating bases modified with reactive groups into nascent polynucleotides are also well-known and commercially available. For example, an isolated polynucleotide can be phosphorylated at the 5'-terminus with phosphorokinase and this phosphorylated polynucleotide covalently attached to an amino-activated substrate through a phosphoramidate or phosphodiester linkage. Other methods will be apparent to those of skill in the art.

In one convenient embodiment, pre-synthesized polynucleotides, modified at their 3'- or 5'-termini with a primary amino group, are conjugated to a carboxy-activated substrate. Chemistries suitable for forming carboxamide linkages between carboxyl and amino functional groups are well-known in the art of peptide chemistry (see, e.g., Atherton & Sheppard, Knorr *et al.*, 1989, Tet. Lett. 30(15):1927-1930; Bannworth & Knorr, 1991, Tet. Lett. 32(9):1157-1160; and Wilchek *et al.,* 1994, Bioconjugate Chem. 5(5):491-492; Solid Phase Peptide Synthesis, 1989, IRL Press, Oxford, England and Lloyd-Williams *et al*., Chemical Approaches to the Synthesis of Peptides and Proteins, 1997, CRC Press, Boca Raton, FL and the references cited therein). Any of these methods can be used to conjugate amino-modified polynucleotides to a carboxy-activated substrate.

Whether synthesized directly on the activated substrate or immobilized on the activated substrate after synthesis or isolation, the polynucleotides can optionally be spaced away from the substrate by way of one or more linkers. As will be appreciated by those having skill in the art, such linkers will be at least bifunctional, i.e., they will have one functional group or moiety capable of forming a linkage with the activated substrate and another functional group or moiety capable of forming a linkage with another linker molecule or the polynucleotides.

Stretches of nucleotides can be interrupted by one or more linker molecules that do not participate in sequence-specific base pairing interactions with a target nucleic acid. The linker molecules may be flexible, semi-rigid or rigid, long or short, charged or uncharged, hydrophobic or hydrophilic, depending on the desired application. A variety of linker molecules useful for spacing one molecule from another or from a solid surface have been described in the art; all of these linker molecules can be used to space regions of immobilized polynucleotides from one another. In an embodiment of this aspect of the invention, the linker moiety is from one to ten, from one to six, alkylene glycol moieties, e.g. ethylene glycol moieties.

In certain circumstances, such linkers can be used to "convert" one functional group into another. For example, an amino-activated substrate can be converted into a hydroxyl-activated substrate by reaction with, for example, 3-hydroxy-propionic acid. In this way, substrate materials which cannot be readily activated with a specified reactive functional group can be conveniently converted into an appropriately activated substrate. Chemistries and reagents suitable for "converting" such reactive groups are well-known, and will be apparent to those having skill in the art.

Linkers can also be used, where necessary, to increase or "amplify" the number of reactive groups on the activated substrate. For this embodiment, the linker will have three or more functional groups. Following attachment to the activated substrate by way of one of the functional groups, the remaining two or more groups are available for attachment of polynucleotides. Amplifying the number of functional groups on the activated substrate in this manner is particularly convenient when the substrate cannot be readily activated with a sufficient number of reactive groups.

Reagents for amplifying the number of reactive groups are well-known and will be apparent to those of skill in the art. A particularly convenient class of amplifying reagents are the multifunctional epoxides sold under the trade name DENACOL.TM. (Nagassi Kasei Kogyo K. K.). These epoxides contain as many as four, five, or even more epoxy groups, and can be used to amplify substrates activated with reactive groups that react with epoxides, including, for example, hydroxyl, amino and sulfonyl activated substrates. The resulting epoxy-activated substrate can be conveniently converted to a hydroxyl-activated substrate, a carboxy-activated substrate, or other activated substrate by well-known methods.

Linkers suitable for spacing biological molecules such as polynucleotides from solid surfaces are well-known in the art, and include, by way of example and not limitation, polypeptides such as polyproline or polyalanine, saturated or unsaturated bifunctional hydrocarbons such as 1-amino-hexanoic acid, polymers such as polyethylene glycol, etc. 1,4-Dimethoxytrityl-polyethylene glycol phosphoramidites useful for forming phosphodiester linkages with hydroxyl groups, as well as methods for their use in nucleic acid synthesis on solid substrates, are described, for example in Zhang *et al.,* 1991, Nucl. 20 Acids Res. 19:3929-3933 and Durand *et al.,* 1990, Nucl. Acids Res. 18:6353-6359. Other useful linkers are commercially available.

The nature and geometry of the solid substrate will depend upon a variety of factors, including, among others, the type of array (e.g., one-dimensional, two-dimensional or three-dimensional) and the mode of attachment (e.g., covalent or non-covalent). Generally, the substrate can be composed of any material which will permit immobilization of the receptor, e.g. polynucleotide, and which will not melt or otherwise substantially degrade under the conditions used to bind the receptor, e.g. hybridize and/or denature nucleic acids. In addition, where covalent immobilization is contemplated, the substrate should be activatable with reactive groups capable of forming a covalent bond with the receptor to be immobilized.

A number of materials suitable for use as substrates in the instant invention have been described in the art. Exemplary suitable materials include, for example, acrylic, styrene-methyl methacrylate copolymers, ethylene/acrylic acid, acrylonitrile-butadienestyrene (ABS), ABS/polycarbonate, ABS/polysulfone, ABS/polyvinyl chloride, ethylene propylene, ethylene vinyl acetate (EVA), nitrocellulose, nylons (including nylon 6, nylon 6/6, nylon 6/6-6, nylon 6/9, nylon 6/10, nylon 6/12, nylon 11 and nylon 12), polycarylonitrile (PAN), polyacrylate, polycarbonate, polybutylene terephthalate (PBT), polyethylene terephthalate (PET), polyethylene (including low density, linear low density, high density, cross-linked and ultra-high molecular weight grades), polypropylene homopolymer, polypropylene copolymers, polystyrene (including general purpose and high impact grades), polytetrafluoroethylene (PTFE), fluorinated ethylene-propylene (FEP), ethylenetetrafluoroethylene (ETFE), perfluoroalkoxyethylene (PFA), polyvinyl fluoride (PVF), polyvinylidene fluoride (PVDF), polychlorotrifluoroethylene (PCTFE), polyethylenechlorotrifluoroethylene (ECTFE), polyvinyl alcohol (PVA), silicon styreneacrylonitrile (SAN), styrene maleic anhydride (SMA), and glass.

Other exemplary suitable materials for use as substrates in the present invention include metal oxides. Metal oxides provide a substrate having both a high channel density and a high porosity, allowing high density arrays comprising different first binding substances per unit of the surface for sample application. In addition, metal oxides are highly transparent for visible light. Metal oxides are relatively cheap substrates that do not require the use of any typical microfabrication technology and, that offers an improved control over the liquid distribution over the surface of the substrate, such as electrochemically manufactured metal oxide membrane. Metal oxide membranes having through-going, oriented channels can be manufactured through electrochemical etching of a metal sheet. Metal oxides considered are, among others, oxides of tantalum, titanium, and aluminum, as well as alloys of two or more metal oxides and doped metal oxides and alloys containing metal oxides. The metal oxide membranes are transparent, especially if wet, which allows for assays using various optical techniques. Such membranes have oriented through-going channels with well controlled diameter and useful chemical surface properties. Patent application EP-A-0 975 427 is exemplary in this respect.

Accordingly, the present invention relates to a method as described herein, wherein said microarray is a flow-through microarray.

Accordingly, the present invention relates to a method as described herein, wherein said substrate is a porous substrate.

Accordingly, the present invention relates to a method as described herein, wherein said substrate is an electrochemically manufactured metal oxide membrane.

Accordingly, the present invention relates to a method as described herein, wherein said substrate comprises aluminum oxide.

The substrate may be in the form of beads, particles, sheets, or membranes and may be permeable or impermeable, depending on the type of array. For example, for linear or three-dimensional arrays the substrate may consist of bead or particles (such as conventional solid phase synthesis supports), fibers (such as glass wool or other glass or plastic fibers), glass or plastic capillary tubes, or metal oxide membranes. For two-dimensional arrays, the substrate may be in the form of plastic or glass sheets in which at least one surface is substantially flat.

The detection of the reporter is indicative for the presence, amount and/or integrity of the analyte. Thus, it is important that the efficiencies of the binding between analyte and receptor, as well as reporter and internal reference are substantially similar. Similarly, it is important that the detection of complexed analyte and receptor, as well as complexed reporter and internal reference are substantially similar.

Use of the arrays of the present invention contemplates the use of reporter polynucleotides and/or analyte nucleic acids that are capable of generating a signal when appropriately bound, e.g. hybridized, to the array.

The signal generated by the internal reference is measured or determined by means of a binding reaction, for example, a hybridisation reaction, with a labeled reporter. The signal generated by the binding of the reporter to the internal reference is preferably distinguishable from the signal generated by the binding of the analyte to the receptor.

Depending on the particular assay protocol with which the subject analyte and reporter nucleic acids are employed, the analyte and reporter nucleic acids may be labeled with the same label, such that the analyte and reporter cannot be distinguished from one another, or the analyte and reporter nucleic acids may be differentially labeled, such that the two sets are readily and/or simultaneously distinguishable from each other.

As such, in certain embodiments, the analyte and reporter nucleic acids are differentially labeled. By "differentially labeled" is meant that the reporter and analyte nucleic acids are labeled differently from each other such that they can be simultaneously distinguished from each other. For example, where one has reporter nucleic acids and analyte nucleic acids, each reporter nucleic acid in the sample will be labeled with the same first label and each analyte nucleic acid in the sample will be labeled with the same second label that is different and distinguishable from the first label. Likewise, where two different sets of reporter nucleic acids are employed in the method, each reporter nucleic acid in the second set will be labeled with a third label different and distinguishable from both the first and second label.

Virtually any label that produces a detectable, quantifiable signal and that is capable of being attached to an analyte and/or reporter, e.g. polynucleotides, can be used in conjunction with the arrays of the invention. Suitable labels include, by way of example and not limitation, radioisotopes, fluorophores, chromophores, chemiluminescent moieties, etc. In embodiments where the label is attached to a polynucleotide, the label can be attached to any part of the polynucleotide, including the free terminus or one or more of the bases. Preferably, the position of the label will not interfere with hybridization, detection or other post-hybridization modifications of the labeled polynucleotide. A variety of different protocols may be used to generate the labeled nucleic acids, as is known in the art, where such methods typically rely on the enzymatic generation of labeled nucleic acid using an initial primer and template nucleic acid. Labeled primers can be employed to generate the labeled target. Alternatively, label can be incorporated into the nucleic acid during first strand synthesis or subsequent synthesis, labeling or amplification steps in order to produce labeled target. Label can also be incorporated directly to mRNA using chemical modification of RNA with reactive label derivatives or enzymatic modification using labeled substrates. Representative methods of producing labeled target are disclosed in U. S. Application Serial nos.: 08/859,998; 08/974,298; 09/225,998.

The reporter polynucleotides or analyte nucleic acids may be labeled, for example, by the labels and techniques described supra. Alternatively, they may be labeled by any other technique known in the art. Preferred techniques include direct chemical labeling methods and enzymatic labeling methods, such as kinasing and nick-translation.

A variety of different labels may be employed, where such labels include fluorescent labels, isotopic labels, enzymatic labels, particulate labels, etc. For example, suitable labels include fluorochromes, e.g. fluorescein isothiocyanate (FITC), rhodamine, Texas Red, phycoerythrin, allophycocyanin, 6-carboxyfluorescein (6-FAM), 2', 7'-dimethoxy-4', 5'-dichloro-6-carboxy-fluorescein (JOE), 6-carboxy-X-rhodamine (ROX), 6-carboxy-2', 4', 7', 4,7-hexachloro-fluorescein (HEX), 5-carboxyfluorescein (5-FAM) or N, N, N', N'-tetramethyl-6-carboxy-rhodamine (TAMRA), cyanine dyes, e.g. Cy5, Cy3, BODIPY dyes, e.g. BODIPY 630/650, Alexa542, etc. Suitable isotopic labels include radioactive labels, e.g. ³²P, ³³P, ³⁵S, ³H. other suitable labels include size particles that possess light scattering, fluorescent properties or contain entrapped multiple fluorophores. The label may be a two stage system, where the target DNA is conjugated to biotin, haptens, etc. having a high affinity binding partner, e.g. avidin, specific antibodies, etc. The binding partner is conjugated to a detectable label, e.g. an enzymatic label capable of converting a substrate to a chromogenic product, a fluorescent label, an isotopic label, etc. Similarly, the detection of the binding between analyte and receptor, as well as the reporter and the internal reference can be indirect. In the present invention, indirect detection relates to the detection of a possible interaction between analyte and receptor or the reporter and the internal reference, in which either the analyte and receptor, and/or the reporter and the internal reference are not labeled. For example, the present invention relates to a sandwich assay, in which analyte and the reporter are antibodies, and wherein the analyte and the reporter are from different species.

It is contemplated that the man skilled within the art will be able to adapt the array format of the present invention to his specific needs. For example, the skilled man may adapt the array format such that the binding of the analyte to the receptor can be detected directly, while the binding of the reporter to the internal reference is detected indirectly. Any combination of labels, e.g. first and second labels, first, second and third labels, etc., may be employed for the reporter sets and analyte in a sample, provided the labels are distinguishable from one another. Examples of distinguishable labels are well known in the art and include : two or more different emission wavelength fluorescent dyes, like Cy3 and Cy5, or Alexa 542 and Bodipy 630/650; two or more isotopes with different energy of emission, like ³²P and ³³P; labels which generate signals under different treatment conditions, like temperature, pH, treatment by additional chemical agents, etc., and labels which generate signals at different time points after treatment.

Using one or more enzymes for signal generation allows for the use of an even greater variety of distinguishable labels based on different substrate specificity of enzymes, e.g. alkaline phosphatase/peroxidase.

Accordingly, the present invention relates to a method as described herein, wherein the reporter comprises a label.

Accordingly, the present invention relates to a method as described herein, wherein the analyte is labeled.

Accordingly, the present invention relates to a method as described herein, wherein the label of the analyte and/or reporter is of the enzymatic, fluorescent, phosphorescent or radioactive type.

Accordingly, the present invention relates to a method as described herein, wherein the label of the analyte differs from the label of the internal reference.

Accordingly, the present invention relates to a method as described herein, wherein the label of the analyte is Texas red, and the label of the internal reference is fluorescein.

Accordingly, the present invention relates to a method as described herein, wherein the label of the internal reference is Texas red, and the label of the analyte is fluorescein.

In embodiments employing *in situ* synthesis, a preferred label is a fluorescently labeled nucleic acid synthesis reagent, such as a labeled nucleoside phosphoramidite. The position at which the fluorophore is attached to the nucleoside phosphoramidite will depend on whether the label will be added at the terminal or internal nucleotides of the nascent polynucleotides. When a terminal label is desired, the fluorophore can be conveniently attached to the 5'-hydroxyl. When internal labels are desired, the flurophore is preferably attached to the base, optionally by way of a linker. Methods suitable for making fluorescently-labeled phosphoramidite synthesis reagents are well-known in the art, and are described, for example, in Goodchild, 1990, supra.

The present invention contemplates that molecules used herein, can be molecular beacons. For example, the receptor and/or the internal reference can be molecular beacons, in which case the analyte and/or reporter are target nucleic acids, respectively. Alternatively, the reporter and/or the analyte can be molecular beacons, in which case the internal reference and/or receptor are target nucleic acids, respectively. Another possibility is that the reporter and the receptor are molecular beacons, in which case the internal reference and/or analyte are target nucleic acids, respectively. Molecular beacons are hairpin-shaped molecules with an internally quenched fluorophore whose fluorescence is restored upon binding to a target nucleic acid. The loop portion of the molecular beacon is complementary to a target, whereas the stem is formed by the annealing of complementary arm sequences. A fluorescent label and a quenching group are attached at the respective ends of the molecular beacon. The stem holds these two groups in close proximity to each other, causing the fluorescence of the fluorophore to be quenched by energy transfer. The quenching group is a non-fluorescent chromophore and emits the energy that it receives from the fluorophore as heat. When the molecular beacon encounters a target molecule, the molecualr beacon forms a hybrid that is more stable than the stem. Thus, the molecular beacon undergoes a spontaneous conformational reorganization that forces the stem apart, and causes the fluorophore and the quencher to move away from each other, leading to the restoration of fluorescence which can be detected. Disclosures by Tyagi and Kramer (1996; Nature Biotechnology 14:303-308) and van Beuningen *et al.* (Proceedings of SPIE vol. 4264 (2001) 66-71) are exemplary in this respect. The quenching moiety of the molecular beacon can be combined with a number of different fluorophores. For example, if two fluorophores are employed, these fluorophores may be different, e.g. the fluorophore of the receptor may differ from the fluorophore of the internal reference.

The present invention contemplates the use of nucleic acid aptamers for detection. An aptamer is an oligonucleotide with a unique sequence that folds into a unique secondary and tertiary structure that, in consequence, present a unique binding surface to its ligands. In this regard, the present invention relates also to aptamer beacons.

Molecular and aptamer beacons may be employed in indirect detection, i.e. detection with a molecular or aptamer beacon of an analyte bound to a receptor and/or of a reporter bound to its internal reference.

For embodiments employing immobilization of pre-synthesized polynucleotides, a preferred label is a labeled polynucleotide. The primary sequences of the labeled and unlabeled polynucleotides at a particular spot may be the same or different. In fact, the same labeled polynucleotide may be used at each spot in the array. The only requirement is that the polynucleotide reagents deposited at each spot in the array be "spiked" with substantially the same proportion of labeled polynucleotide.

In an embodiment, the same mixture of labeled polynucleotides is used to spike the polynucleotide reagent deposited at each spot. Using the same mixture of labeled polynucleotides at each spot ensures that the labels at different spots do not induce sequence-specific anomalies in hybridization assays, i.e., it ensures that the labels at each array spot interact similarly with a target nucleic acid in hybridization assays. Moreover, use of the same label at each spot reduces the number of labeled polynucleotides that need to be prepared.

The amount of label used to "spike" the polynucleotide reagent to be deposited at a particular spot is not critical for success. However, the amount used should be sufficient to produce a detectable signal which does not result in a loss of dynamic range when the array is used in an assay.

For use in a hybridization array, the background signals from a polynucleotide array according to the invention are quantified and recorded. The mode of detection will depend on the nature of the label. For fluorescent labels, the background signals can be conveniently quantified by scanning the array with a confocal camera or with a CCD camera, as is well-known in the art.

The array is contacted with a reporter and analyte nucleic acid, which may be labeled or unlabeled, depending on the particular array format, under conditions which discriminate between perfectly complimentary hybrids and hybrids containing one or more mismatches. The actual hybridization conditions used will depend upon, among other factors, the G+C content of the sequence of interest and the lengths of the immobilized polynucleotides comprising the array. Hybridization conditions useful for discriminating between perfect compliments and mismatches for a variety of hybridization arrays have been described in the art. For example, hybridization conditions useful for discriminating complimentary and mismatched hybrids in a variety of applications are described in U.S. Pat. No. 5,525,464 to Drmanac *et al.,* WO 95/09248 and WO 98/31836. A detailed discussion of the theoretical and practical considerations involved in determining hybridization conditions, and including a discussion of the advantages of low-temperature washing steps, may be found in WO 98/31836, particularly pages 50-62. Additional guidance may be found in Harmes and Higgins, Nucleic Acid Hybridization: A Practical Approach, 1985, IRL Press, Oxford, England.

As mentioned above, in practicing the subject methods the analyte and reporter nucleic acids are hybridized to an array, where the target comprising analyte and reporter nucleic acids may be hybridized to the same array or different arrays, where when the analyte and reporter nucleic acids are hybridized to different arrays, all of the different arrays may at least share common arrays, spots or binding substances of receptor and/or internal reference nucleic acids, e.g. they will be identical with respect to their receptor and/or internal reference nucleic acids.

In the above embodiments where the analyte and reporter nucleic acids are hybridized simultaneously to a given array, labeled analyte and reporter nucleic acids are premixed or pooled prior to contact with the array. In an embodiment, mixtures of analyte and reporter nucleic acids have amounts of the analyte and reporter nucleic acids which are sufficient to generate signals that are at least 1.5 fold, usually at least 3 fold and more usually at least 5 fold higher than background signals observed with the array. The relative amounts of the analyte and reporter nucleic acids in the mixture are selected to be sufficient to allow reliable detection of the test sequences complimentary to the respective receptor and internal reference nucleic acid while at the same time allowing complete binding of the reporter nucleic acids with a nofold excess of unbound reporter nucleic acid on the array. The amount of reporter nucleic acid present in the mixture is usually determined by available amount of sample and sensitivity of technology employed in a particular protocol. For example, the amount of reporter nucleic acid present in the mixture ranges from about 0.01-100 µg of nucleic acid, e. g. cDNA, and more usually from about 0.1-10 µg of nucleic acid, e. g. cDNA. In many embodiments, the amount of reporter nucleic acid employed in the hybridization protocol is about the same or less than the amount of analyte nucleic acid that is employed, where less than typically means 10 fold less, usually 100 fold less and more usually 1000 fold less. Of interest are mixtures of labeled nucleic acids that provide for an intensity of signal from each probe nucleic acid in the control detection channel that ranges from about 0.001 to 0.1 %, usually from about 0.001 to 0.01% abundance level.

The reporter and analyte nucleic acids are hybridized to the array(s) by contacting the analyte and reporter nucleic acids with the array(s) under hybridization conditions. By "hybridization conditions" is meant conditions sufficient to promote Watson-Crick hydrogen bonding to occur between the target and probe nucleic acids. The hybridization conditions, such as hybridization time, temperature, wash buffers used, etc. can be altered to optimize the efficient and specific binding of the target sequences. Test target nucleic acids having sequence similarity to the probes may be detected by hybridization under low stringency conditions, for example, at 50 °C and 6xSSC (0.9 M sodium chloride/0.09 M sodium citrate, 1% SDS) and remain bound when subjected to washing at 55 °C in 1 x SSC (0.15 M sodium chloride/0.015 M sodium citrate, 1% SDS). Test target sequences with sequence identity may be determined by hybridization under stringent conditions, for example, at 60 °C or higher and 6xSSC (15 mM sodium chloride/01.5 mM sodium citrate, 1 % SDS). For example, the analyte and reporter nucleis acids have a region of substantial identity to the provided receptor and internal reference sequences on the array, respectively, and bind selectively to their respective receptor and internal reference sequences under stringent hybridization conditions. Other suitable hybridization conditions for various nucleic acid pairs are well known to those skilled in the art and reviewed in Sambrook et al., 1989 (see infra), and in PCT WO 95/2144.

Analysis of the differences in signal generated by two or more sources may be carried out by using multiple arrays with the same or similar receptor and internal reference compositions, each array for each set of analyte and reporter nucleic acids. Each array is then hybridized with labeled reporter target nucleic acids and labeled analyte nucleic acids. For instance, the labeling efficiency and amount of analyte sequences and reporter sequences is approximately equivalent between arrays, e. g. an equal amount of labeled analyte nucleic acids is used to hybridize to each array. This is not essential, however, since hybridization of the set of labeled reporter nucleic acids functions as an independent internal control for each probed array.

Levels of hybridization of reporter RNA to the binding substances can be standardized by comparing the hybridization signal of the reporter with internal reference sequences on each array.

### Differences in hybridization of the predefined reporter sequences to the predefined internal references allows a comparison of relative hybridization levels between arrays

Following hybridization, non-hybridized labeled nucleic acid is removed from the substrate, conveniently by washing, generating a pattern of hybridized nucleic acid on the substrate surface. A variety of wash solutions and protocols are known to those of skill in the art and may be used. See Sambrook, Fritsch & Maniatis, Molecular Cloning: A Laboratory Manual (Cold Spring Harbor Press) (1989).

If the analyte and/or reporter is labeled, the array can be scanned or otherwise analyzed for detectable assay signal, and the signal from each labeled spot, or alternatively from all spots, quantified. Only those spots where binding, e.g. hybridization, occurred will produce a detectable assay signal. The resultant hybridization patterns of labeled nucleic acids may be visualized or detected in a variety of ways, with the particular manner of detection being chosen based on the particular label of the target nucleic acid, where representative detection means include scintillation counting, autoradiography, fluorescence measurement, colorimetric measurement, light emission measurement, light scattering and the like (see above).

Following detection, determination or visualization, the binding, e.g. hybridization, patterns generated by analyte and reporter, for example analyte and reporter nucleic acids, may be compared to identify differences between the signals. Where arrays in which each of the different receptor corresponds to a known gene are employed, differences in signal intensity can be related to a different analyte concentration of a particular gene.

The comparison of the intensity of the signal resulting from the binding of an analyte nucleic acid to a receptor can be compared to the intensity of the signal resulting from the binding of the corresponding reporter sequence to the internal reference sequence, and the measurement converted to a relative quantitative nucleic acid concentration for that analyte sample. The relative quantitative nucleic acid levels of the analyte can be compared within and between arrays to identify, determine or confirm differential expression of genes in particular samples.

If each spot in the array contains the same quantity of immobilized polynucleotide, in theory, the intensity of the assay signal at each spot will be proportional to the extent of hybridization at that spot. For example, spots containing perfectly complementary hybrids are expected to produce more intense assay signals than spots containing mismatched hybrids. In practice, however, differences in signal intensities between different spots may instead be due to differences in the amounts of polynucleotide immobilized at the respective spots or amounts of analytes due to sample preparation.

Because each spot in the arrays of the invention contains an amount of an internal reference proportional to the amount of receptor immobilized at the particular spot, the assay signals obtained from the arrays of the invention can be normalized. As a consequence, signal intensities from spots within a single array, within spots, or across multiple arrays, can be directly compared, without regard to the fidelity of the particular array synthesis or the sample preparation.

The method by which the signals are normalized will depend upon whether the reporter or background signals are the same as the assay signals, such as where the reporter and analyte are labeled with the same fluorophore. In this embodiment, a normalized signal of a particular spot is defined by (la -lb)/lb, where la is the intensity of the assay signal of the spot (e.g. intensity of the spot after hybridization) and Ib is the intensity of the background signal of the spot (e.g. the intensity of the spot before hybridization).

In embodiments where the reporter and assay signals are different, e.g. where the reporter and the analyte are differently labeled, e.g. with different fluorophores, the normalized signal for a spot is described by la /lb, where la is the intensity of the assay signal of the analyte and Ib is the intensity of the reporter signal of the same spot.

Accordingly, the present invention relates to a method for the normalization of an array comprising the steps of:
(i) immobilizing onto said array a binding substance comprising a receptor and a predetermined amount of an internal reference, and,
(ii) determining the signal generated by said internal reference by means of a reporter molecule which selectively binds to said internal reference.

While the array is illustrated utilizing labeled analyte and reporter nucleic acids, those of skill in the art will recognize that the arrays of the invention are also useful in assays employing unlabeled target nucleic acids. The only requirement is that some component of the particular assay generate a detectable signal at spots where binding, e.g. hybridisation, occurs.

The subject methods find use in, among other applications, standardization of differential gene expression assays. Thus, one may use the subject methods in the differential expression analysis of: (a) diseased and normal tissue, e. g. neoplastic and normal tissue, (b) different tissue or tissue types; (c) developmental stage; (d) response to external or internal stimulus; (e) response to treatment; and the like. The methods of the subject invention therefore find use in broad scale expression screening for drug discovery, diagnostic and research, such as the effect of a particular active agent on the expression pattern of genes in a particular cell, where such information can be used to reveal drug toxicity, carcinogenicity, etc., environmental monitoring, disease research and the like. A number of different tasks can be accomplished with the subject invention, which tasks include, but are not limited to: detecting relative hybridization of target sequences, calibrating a hybridization assay, harmonizing data between hybridization assays, and testing reagents used in a hybridization assay. The subject methods in which control and test sets of target nucleic acids are employed can also be used in the generation of gene expression databases, as the data generated from the subject methods are relative quantitative, reflect relative RNA concentration rather than intensity of signal, and are independent of the type of array. Each of these different aspects of the invention is discussed separately below.

The methods of the present invention are useful in detecting relative levels of hybridization of different genes in a sample by providing a set of internal hybridization controls, i.e. the reporter. Since the reporter nucleic acids are of a known sequence, in a known quantity, and of a known specific activity (where in an exemplary embodiment the reporter and analyte are labeled with the same specific activity), the level of hybridization of the reporter nucleic acids can be used to determine the level of expression of each gene in a test sample based on its level of binding to a receptor sequence. The provision that each sample has its own internal control (reporter) also allows for the detection of potential expression differences between samples and differences in binding affinities between receptor sequences, both on a single array and between arrays. Thus, the intensity level of hybridization of a reporter sequence can be used to calculate the expression level of a gene in a sample based upon the intensity of the analyte hybridization to the corresponding receptor sequence.

The methods of the subject invention also find use in the calibration of hybridization assays. Using known concentrations of receptor nucleic acid, analyte nucleic acids, internal reference nucleic acids and reporter nucleic acids allows one to optimize the hybridization conditions for a particular use, such as increasing stringency to allow better detection of nucleic acids with some level of sequence homology (e.g. differential expression between genes from a single family or alternative splice forms for the same gene). The use of the internal standards of the method of the subject invention allows hybridization, labeling procedures, and the like to be optimized for a particular use, which is especially valuable for standardization of large scale of hybridization assays, such as high throughput screening of biological samples. Optimization thus means that one can change hybridization conditions in order to achieve maximal intensity of specific hybridization signals with complimentary probe sequences and minimal level of non-specific hybridization with non-complementary probe sequences.

The methods of the subject invention also find use in the harmonization of data between hybridization assays, thus allowing for a direct comparison of expression levels despite potential differences due to variables such as differences in hybridization conditions, differences in sample preparation and even between different types of arrays, differences in quality and performance within and between different arrays, differences in specific activity of the labeled target sequences, and the like. Because each hybridization assay has its internal control for at least a subset of the probe sequences on the array, the data can be compared using ratios of the intensity of the reporter nucleic acids and the intensity of the analyte nucleic acids. Thus, the use of simple mathematical formulations to correct for differences between assays allows the levels of gene expression in these different assays to be adjusted to the same level and then compared in a biologically relevant fashion.

The methods of the present invention are also useful in determining the efficacy of hybridization reagents. Such reagents may be, for example, new reagents, e.g. different buffer solutions for prehybridization and hybridization, or established reagents, e.g. a new batch of a known, commercially available reagent. The internal control of the methods of the subject invention provide for two levels of quality assurance upon testing the reagents, basically providing an extra control for determining the efficacy of a reagent in a single hybridization. Efficiency means maximum specific signal with minimal level of non-specific signal and background binding to solid surface. Other parameters such as temperature, buffer composition, length of hybridization and/washing times, etc., may be optimized using calibration controls. Also, the same calibration reporter nucleic acids can be used routinely to test and calibrate detection equipment to expected level intensity of signals, thus limiting variability due to functionality of the equipment; variation due to data generated in different labs, or at different times, or even using different types of arrays.

Accordingly, the present invention relates to a method as described herein for use in expression profiling assay, genotyping, sequence determination by hybridization, gene quantitation, gene abnormality analysis (Multiplex Amplifiable Probe Hybridisation, MAPH), PCR, NASBA, or TYRAS.

Accordingly, the present invention relates to the use of an array for normalisation of analyte variation, wherein said array comprises a substrate with predefined regions, wherein each binding substance immobilized at a predefined region of said substrate comprises a receptor and a predetermined amount of an internal reference, wherein the signal generated by said internal reference is determined by means of a reporter molecule and wherein said reporter molecule selectively binds to said internal reference.

Accordingly, the present invention relates to the use of an array in a method as described herein.

Accordingly, the present invention relates to an array for use in a method as described herein, wherein said array comprising a substrate with predefined regions, wherein each binding substance immobilized at a predefined region of said substrate comprises a receptor and a predetermined amount of an internal reference, wherein the signal generated by said internal reference is determined by means of a reporter molecule and wherein said reporter molecule selectively binds to said internal reference.

Accordingly, the present invention relates to an array comprising a substrate with predefined regions, wherein each binding substance immobilized at a predefined region of said substrate comprises a receptor and a predetermined amount of an internal reference, wherein the signal generated by said internal reference is determined by means of a reporter molecule and wherein said reporter molecule selectively binds to said internal reference.

Tyras is a method for amplifying RNA by creating, in a non-specific manner, multiple RNA copies starting from nucleic acid containing starting material comprising a pool of mRNAs each mRNA comprising a poly-A tail, wherein the material is contacted simultaneously with an oligonucleotide comprising an oligo-dT sequence, the sequence of a promoter recognized by a RNA polymerase and a transcription initiation region which is located between the oligo-dT sequence and the sequence of the promoter, and further with an enzyme having reverse transcriptase activity, an enzyme having RNase H activity and an enzyme having RNA polymerase activity and the necessary nucleotides and the resulting reaction mixture is maintained under the appropriate conditions for a sufficient amount of time for the enzymatic processes to take place. This will lead to the formation of multiple anti-sense RNA copies of the mRNAs present in the reaction mixture. Tyras does not involve the production of cDNA intermediates; RNA is copied directly from the mRNA present in the material under investigation. Tyras does not need a cDNA as a basis for the amplification of the RNA. The RNA is synthesized by an RNA polymerase, directly from the mRNA template. The activity of the RNA polymerase is independent from any secondary structures present in the mRNA and thus there are no differences in the way the different mRNAs are amplified depending on structures in the mRNAs. The copies made represent the original mRNA population as present in the starting material. The oligonucleotides used with Tyras comprise an oligo-dT sequence which will hybridize to the poly-adenylated tail at the 3' end of the mRNAs. The oligonucleotides further comprise the sequence of a promoter recognized by an RNA polymerase and a transcription initiation region which is located between the oligo-dT sequence and the sequence of the promoter. The promoter may be the promoter for any suitable RNA polymerase. Examples of RNA polymerases are polymerases from *E*. *coli* and bacteriophages T7, T3 and SP6. In this respect, WO 99/43850 by Pam Gene is exemplary.

The present invention also provides kits for performing the subject array-based hybridization assays. The subject kits at least include reporter nucleic acids, as defined above, or a precursor thereof. By "nucleic acid precursor" is meant any nucleic acid from which with the control set may be prepared, e.g. a set of RNAs encoding the nucleic acids of the control set, plasmids containing nucleic acids for generation of the control set, and the like.

Labeled cDNA can be derived from these precursors by enzymatic synthesis, or oligonucleotides chemically synthesized based on sequence information of these precursors. The kits may contain RNAs that recognizes each probe composition on an array, and such RNAs may be pre-labeled, may be labeled for use with the analyte nucleic acids, or may be converted to labeled cDNA for hybridization. Kits of the present invention may also contain cDNA or oligonucleotides that selectively bind to the receptor compositions of the array to be screened. The cDNAs or oligonucleotides may be pre-labeled, or may be labeled by the user through any convenient protocol, such as the protocol used to generate the labeled reporter nucleic acids. A kit containing a set of control target RNAs may further contain oligonucleotides for the production of cDNA. In an exemplary embodiment, these oligonucleotides are gene specific primers, particularly gene specific primers that have sequence identical to those that were used in the production of the receptor compositions on the array to be used in the particular assay. In another embodiment, primers can be oligo dT or random primer, if these primers are used for making test sample target.

Kits for carrying out differential gene expression analysis assays are contemplated. Such kits according to the subject invention will at least comprise the subject sets of nucleic acids, e.g. receptors and internal references. The kits may further comprise one or more arrays corresponding to the set of reporter nucleic acids.

The kits may further comprise one or more additional reagents employed in the various methods, such as: primers for generating target nucleic acids; dNTPs and/or rNTPs, which may be either premixed or separate; one or more uniquely labeled dNTPs and/or rNTPs, such as biotinylated or Cy3 or Cy5 tagged dNTPs; or other post synthesis labeling reagents, such as chemically active derivatives of fluorescent dyes, enzymes such as reverse transcriptases, DNA polymerases, RNA polymerases and the like; various buffer mediums, e.g. hybridization and washing buffers; prefabricated probe arrays; labeled probe purification reagents and components, like spin columns, etc.; signal generation and detection reagents, e.g. streptavidin-alkaline phosphatase conjugate, chemifluorescent or chemiluminescent substrate; and the like.

In addition to the sets of nucleic acids, arrays and other components described above in the general description of kits, the assay kit may further include a set of gene specific primers that are employed to generate labeled analyte nucleic acids. In many embodiments, the set of gene specific primers will be the same primers used to generate the polynucleotide receptors that are present on the array to be screened.

Accordingly, the present invention relates to a device or kit comprising a flow-through based array as described herein.

Accordingly, the present invention relates to the use of a device or kit as described herein, in expression profiling assay, genotyping, sequence determination by hybridization, gene quantitation, gene abnormality analysis (MAPH), PCR, NASBA, or TYRAS.

Accordingly, the present invention relates to the use of a reporter molecule for the manufacture of or the incorporation into a device or kit as described herein.

Accordingly, the present invention relates to the use of an internal reference for the manufacture of or the incorporation into a kit or device as described herein.

Accordingly, the present invention relates to a method for correlating variation in analytes, comprising:
- providing at least two analytes, wherein each analyte is identified according to the method of the present invention,
- comparing the values of the normalised analytes as defined in the present invention, whereby variation in analytes is correlated.

Accordingly, the present invention relates to a method of generating a report that correlates analyte variation determined by a method according to the present invention.

Accordingly, the present invention relates to a computer system comprising data obtained according to a method, assay, array or kit of the present invention.

Before the subject invention is described further, it is to be understood that the invention is not limited to the particular embodiments of the invention described herein, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

The following examples are offered by way of illustration and not by way of limitation.

### SHORT DESCRIPTION OF THE FIGURES

**Figure 1:** Fluorophore for the reporter probe
   (A) overview of the array; (B) signal detected with an NBB filter g5f20 (Narrow band blue filter); (C) signal detected with a WIGfilterg0f1_125 (Super wide band green).
**Figure 2:** IRP / receptor ratio optimisation
   (A) array overview; (B) sample fluoresceine-signal with a Narrow band blue filter, after 30 minutes at 770ms integration time; (C) IRP Texas red signal with a Wide band green filter, after 30 minutes at 440ms integration time.
**Figure 3:** Normalisation of PamChip
   (A) array overview; (B) sample signal with Narrow band blue filter, after 30 minutes at 440ms integration time (inhomogeneous by illumination errors); (3C) IRP signal with Wide band green filer, after 30 minutes at 27.5ms integration time (Inhomogeneous by illumination errors); (D) sample signal with Narrow band blue filter, after 30 minutes at 770ms integration time; (E) total illuminated area with an indication of an air bubble on the left part of the image, corresponding to (D); (F) IRP signal with Wide band green filter, after 30 minutes at 200ms integration time; (G) total illuminated area with an indication of the air bubble on the left part of the image, corresponding to (F).

### EXAMPLES

### Example 1. Materials

Detections were performed utilising fluorescent microscopy (Olympus, Tokyo Japan).

Oligonucleotides were prepared and coupled to the substrate as previously described in PCT/EP98/04938. A non-human plant virus sequence from the Potato Leafroll RNA Virus (PLRV)-S2 sequence was used as internal reference IRP; (see Klerks *et al.* J. Vir. Methods 93 (2001)115-125).

### Oligonucleotide sequences:

- -IRP:: PLRV-s2 (SEQ ID NO: 1; tgcaaagtatcatccctccag) (5'activated)
- -Rho:: Reporter probe, 5'-Rhodamine labelled comPLRV_rho
(SEQ ID NO: 2; ctggagggatgatactttgca)
- -Rox:: Reporter probe 5'-ROX labelled comPLRV_rox
(SEQ ID NO: 3; ctggagggatgatactttgca)
- -TxR:: Reporter probe 5'-Texas Red labelled comPLRV_tex
(SEQ ID NO: 4; ctggagggatgatactttgca);
- -F2:: Target sequence 5'-fluorescein labelled F2,
(SEQ ID NO: 5; TCC TTT TCC AGT TCT GTA CAA)
- -R: REF1(S2+F), (5'-FAM labelled) designated as R
(SEQ ID NO: 6; catgtatcgaggataaatgaag)
- -: HIVpol7p41-3, -5, -6, 10, -16, -18, -20, -22, -23, corresponding to SEQ ID NOs: 7, 9, 10, 11, 12, 13, 14, 16 and 17, respectively (see Table 2)

### Example 2. Fluorophore for the reporter probe

In order to simultaneously distinguish reporter binding to the internal reference (IRP) and analyte binding to receptor, respectively, reporter and analyte should be differentially labeled. Below an experiment is given with PamGene microarray spots of 300 pL of Rhodamine (Rho), ROX (Rox) and Texas Red (Tx) labelled oligonucleotides (each 10µM) and Fluorescein labelled oligonucleotide (F2) of 1µM.

The experimental set up was essentially as described in WO 99/0226.

In short, oligonucleotide probes were covalently coupled to the Anopore membranes using 3-aminopropyl triethoxysilane (APS) as a linker between the alumina and the oligonucleotide.

After rinsing with water, the membranes were dried and immersed in a 0.25% (v/v) solution of APS in water for 2 hours. Excess APS was removed by rinsing with water. After drying at 120°C at reduced pressure the membranes were stored. Amino group concentration due to the coupling of the APS molecules was typical 2-3 µmol/m².

Before coupling, the amino group terminated oligo nucleotides were activated by reaction with disuccinimidyl suberate (DSS, see eg. PIERCE BV, lmmunotechnology Catalog & Handbook, 1990). The resulting succinimidyl group at the end of the oligonucleotide was used for coupling to the APS activated membrane. Coupling with oligonucleotide solution on an Anopore membrane during 60 minutes resulted in a coupling yield of 1 x 10⁻¹⁰ mol/m² oligonucleotides.

For detection, fluorescent microscopy was utilised as described in Example 1 (Olympus, Tokyo Japan).

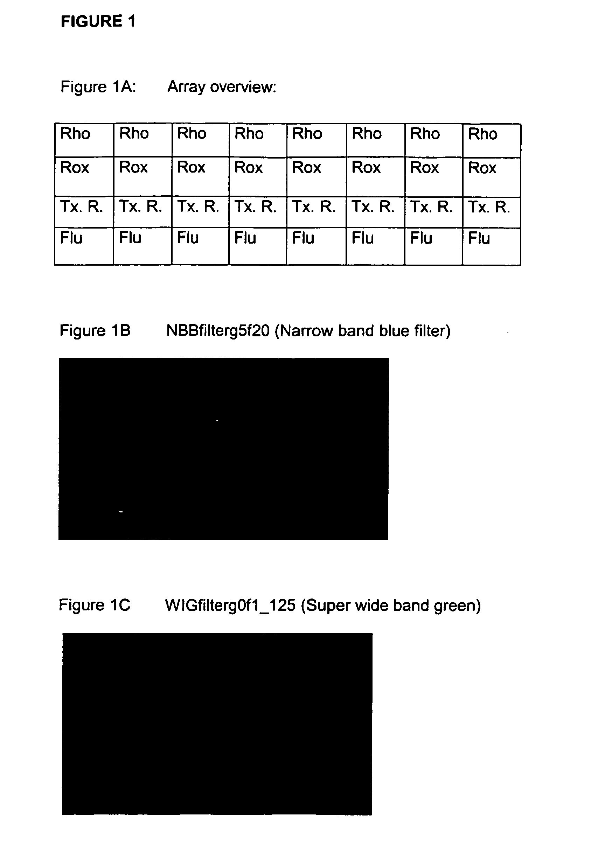

Figure 1A depicts the overview of the array. Figure 1 B depicts the signal resulting from using an NBB filter g5f20 (Narrow band blue filter). Figure 1C depicts the signal resulting from using a WIGfilterg0f1_125 (Super wide band green).

In order to minimise cross-talk between the fluorophores, the fluorophore used for the reporter probe should preferably have a distinct excitation and emission profile as compared to the fluorophore used at the target (analyte). As fluorophore of the reporter probe preferentially Texas Red > ROX > Rhodamine should be used in combination with a fluorescein sample fluorophore (analyte).

### Example 3. IRP / receptor ratio optimisation

The IRP (PLRV-s2; SEQ ID NO: 1) was mixed in different concentrations with the subject receptor (HIVpol7p41-4; SEQ ID NOs: 8), according to Table 1. The mixtures were subsequently covalently coupled as outlined in Example 2.

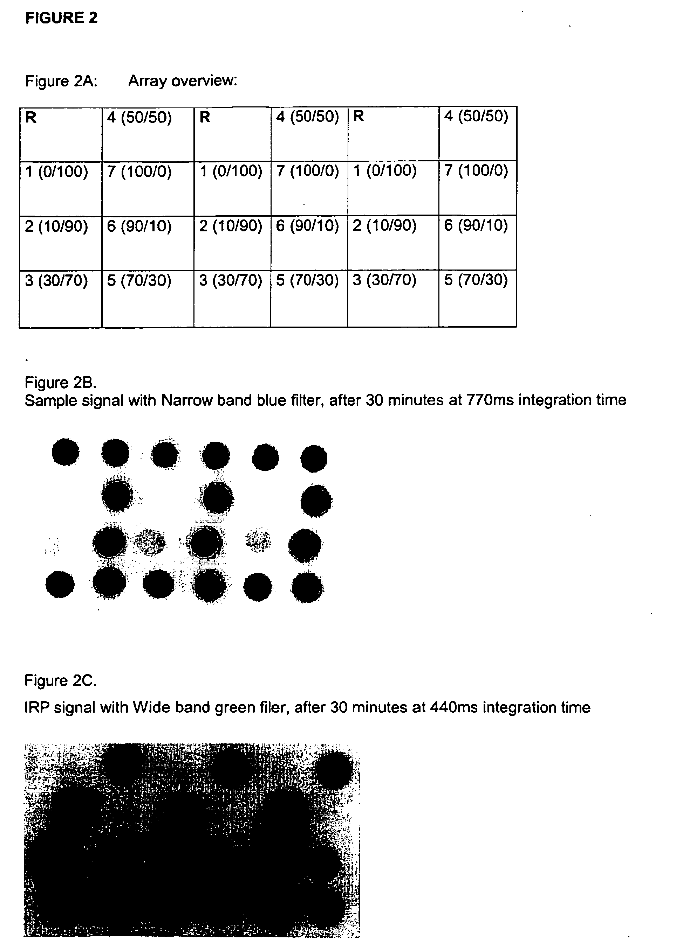

Different ratio's of the IRP and receptor were spotted in three-fold within one array, as depicted in Figure 2A. Next, the micorarray was hybridised with a mixture of Tx.R. and the fluoresceine labeled HIV oligo F2, i.e. 20 µl of 1nM reference probe comPLRV-Texas red (Tx.R.; analyte) and 20µl of 1nM reference probe HIV-oligo F2 (reporter) in 0.6x SSPE at 45°C for 30 minutes at 2 pumping steps per minute with subsequent washing step with 0.6x SSPE at 45°C. The Fluoresceine-signal (by F2) was determined with a Narrow band blue filter (Figure 2B), while the Texas red signal (by Tx.R.) was determined with a Wide band green filter (Figure 2C).

**Table 1: Different ratio's between receptor HIVpol7p41-4 and IRP PLRV-s2.**

| | **Sample HIVpol7p41-4** | **IRP PLRV-s2** |
|---|---|---|
| **1** | 0% | 100% |
| **2** | 10% | 90% |
| **3** | 30% | 70% |
| **4** | 50% | 50% |
| **5** | 70% | 30% |
| **6** | 90% | 10% |
| **7** | 100% | 0% |

Interference from the signal resulting from the IRP-reporter with the signal resulting from the receptor-reporter is not detectable. Furthermore, interference between the signal resulting from the analyte binding to the receptor and the signal resulting from the IRP binding to the reporter is not detectable. An amount of 10-90% IRP added to the receptor may be used.

### Example 4. Normalisation of PamChip

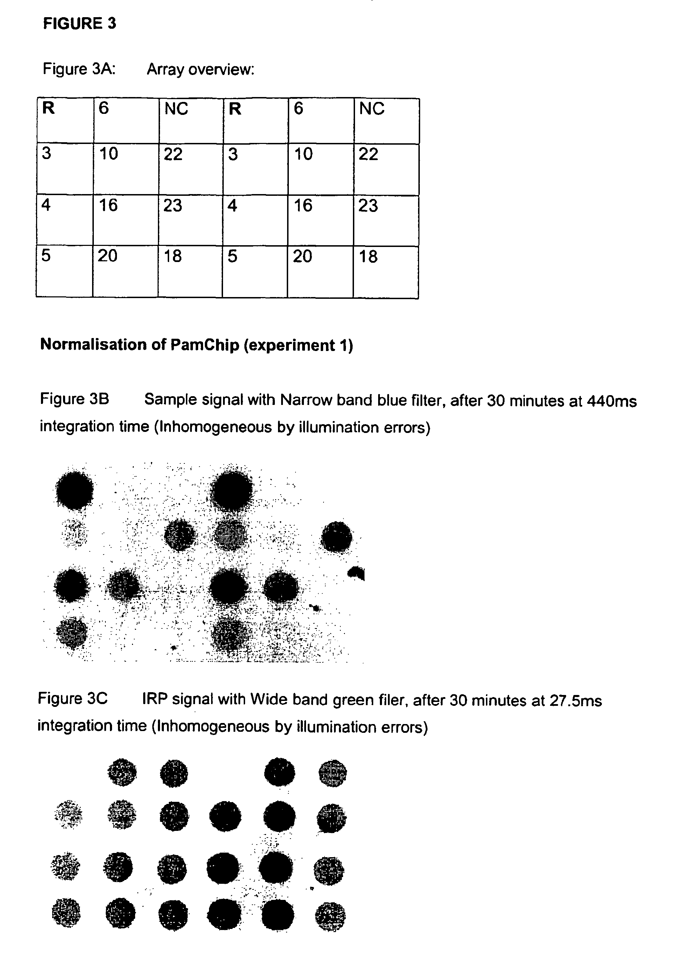

An array with 11 different specific receptors, i.e. probes, each having a different amount of mismatches to the fluorescein labeled target oligo (analyte) was made. The specific receptors are depicted in Table 2. Before spotting, these receptor probes were mixed with the IRP PLRV-s2 with a per cent ratio of 70/30. An overview of the array is depicted in Figure 3A.

Hybridisation as outlined in Example 3, was performed using the same conditions as outlined above.

Application of the IRP normalisation was performed on deliberately inhomogeneous illuminations of arrays. The two methods used were based on an inhomogeneous light source illumination and inhomogeneous signals by addition of an air bubble below the array. Image and spot signal intensity determination was done with Array-Pro (MediaCybernetics).

**Table 2: Information of specific receptors (probes) spotted on the array**

| SEQ ID No | name oligo | Sequence | mismatches with target |
|---|---|---|---|
| 7 | HIVpol7p41-3 | 5'- TTG TAC AGA GAT GGA AAA GGA | 2 |
| 8 | HIVpol7p41-4 | 5'- TTG TAC AGA ACT GGA AAA GGA | 0 |
| 9 | HIVpol7p41-5 | 5'- TTG TGC AGA AAT GGA AAA GGA | 2 |
| 10 | HIVpol7p41-6 | 5'- TTG TAC AGA AAT GGA AAA AGA | 2 |
| 11 | HIVpol7p41-10 | 5'- TTG CAC AGA AAT GGA AAA GGA | 2 |
| 12 | HIVpol7p41-16 | 5'- TTG TAC AGA ACT GGA GAA GGA | 1 |
| 13 | HIVpol7p41-18 | 5'- TTG TAA AGA GAT GGA ACA GGA | 4 |
| 14 | HIVpol7p41-20 | 5'- TTG TGC AGA TAT GGA AAA GGA | 3 |
| 15 | HlVpol7p41-21 | 5'- TTG TGC ATT TAT GGA GGA GGA | 7 |
| 16 | HlVpol7p41-22 | 5'- TTG TAC AGA ATT GGA AAA GGA | 1 |
| 17 | HlVpol7p41-23 | 5'- TTG TTT AGA AAT GGA AAA GGA | 3 |
| 18 | flu labelled target | 5'- TCC TTT TCC AGT TCT GTA CAA | |

| | | | |
|---|---|---|---|
| NC = negative control, complete different sequence | | | |
| R= reference, fluorescein labeled oligo for positioning | | | |

**Table 3 Results of the first series of experiments**

| **Array Position** | **Oligo** | **Signal** | | **Sample/IRP Nomalised** | **CV%** | |
|---|---|---|---|---|---|---|
| | | **Sample** | **IRP** | | **Sample** | **Normalised** |
| 1-1:2 | 6 l | 1.5 | 27.9 | 1.5 | 47% | 43% |
| 1-1:5 | 6 | 3.0 | 30.0 | 2.8 | | |
| 1-1:3 | NC l | 0.0 | 27.1 | 0.0 | | |
| 1-1:6 | NC | 0.1 | 28.5 | 0.1 | | |
| 1-2:1 | 3 l | 8.2 | 26.2 | 8.7 | 27% | 15% |
| 1-2:4 | 3 | 11.9 | 30.8 | 10.8 | | |
| 1-2:2 | 10 l | 2.3 | 26.2 | 2.4 | 45% | 40% |
| 1-2:5 | 10 | 4.4 | 28.4 | 4.3 | | |
| 1-2:3 | 22 l | 16.3 | 27.3 | 16.6 | 31% | 23% |
| 1-2:6 | 22 | 25.3 | 30.5 | 23.3 | | |
| 1-3:1 | 4 l | 28.5 | 25.1 | 31.7 | 19% | 3% |
| 1.3:4 | 4 | 37.5 | 31.5 | 33.3 | | |
| 1-3:2 | 16 l | 16.1 | 27.5 | 16.4 | 25% | 15% |
| 1-3:5 | 16 | 23.0 | 31.9 | 20.2 | | |
| 1-3:3 | 23 l | 0.6 | 24.1 | 0.6 | | |
| 1-3:6 | 23 | 0.4 | 25.6 | 0.4 | | |
| 1-4:1 | 5 l | 13.9 | 24.0 | 16.2 | 12% | 8% |
| 1-4:4 | 5 | 16.4 | 31.9 | 14.4 | | |
| 1-4:2 | 20 l | 0.7 | 27.5 | 0.7 | 25% | 16% |
| 1-4:5 | 20 | 1.0 | 31.3 | 0.9 | | |
| 1-4:3 | 18 l | 1.2 | 26.6 | 1.3 | | |
| 1-4:6 | 18 | 0.8 | 25.4 | 0.9 | | |
| | | | 28.0 | | 29% | 21% |

Normalization was done by (Net Sample signal / Net IRP) / Average IRP, wherein the Net Sample signal is the signal resulting from the analyte to the receptor. The effects of normalization were expressed in terms of average variation between duplicate spots.
**4.1** The first series of experiments are depicted in Figure 3B, relating to sample signal with Narrow band blue filter, after 30 minutes at 440ms integration time (Inhomogeneous by illumination errors), and Figure 3C, relating to the IRP signal with Wide band green filter, after 30 minutes at 27.5ms integration time (inhomogeneous by illumination errors). The results of the first series of experiments are summarized in Table 3.

Normalisation reduces the variation between spots from 29±12% to 21±14%. Normalisation with the IRP has lead to a 33% lower variation between duplicates.
**4.2** The second series of experiments relates to environmental influences on the picture, resulting in bad duplicates. In this case, an air bubble was created under the slide. The results are depicted in Figures 3D-3G.

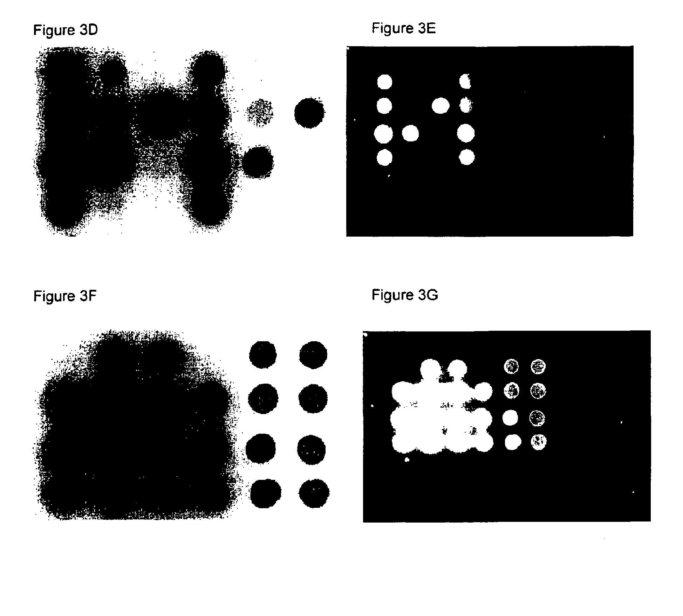

Figure 3D depicts the sample signal with Narrow band blue filter, after 30 minutes at 770ms integration time. Figure 3E, which corresponds to Figure 3D, demonstrates a total illuminated area with an indication of an air bubble on the left part of the image.

Figure 3F depicts the IRP signal with Wide band green filter, after 30 minutes at 200ms integration time. Figure 3G, which corresponds to Figure 3F, depicts the total illuminated area with an indication of the air bubble on the left part of the image.

Note, during imaging of the arrays of the first end second series of experiments, the air bubble shifter slightly, less than 50 µm, between the two images taken on the array.

The results of the second series of experiments are summarized in Table 4.

Normalisation reduces the variation between duplicates from 34±21% to 15±6%. Normalization with the IRP has lead to a 50% lower variation between duplicates.

**Table 4 Results of second series of experiments**

| **Array Position** | **Oligo** | **Signal** | | **Sample/IRP Nomalised** | **CV%** | |
|---|---|---|---|---|---|---|
| | | **Sample** | **IRP** | | **Sample** | **Normalised** |
| 1-1:2 | 6 l | 16.6 | 87.6 | 12.9 | 48% | 17% |
| 1-1:5 | 6 | 8.2 | 34.1 | 16.3 | | |
| 1-1:3 | NC l | 0.0 | 68.9 | 0.0 | | |
| 1-1:6 | NC | 0.0 | 36.7 | 0.0 | | |
| 1-2:1 | 3 l | 75.6 | 123.2 | 41.7 | 15% | 22% |
| 1-2:4 | 3 | 61.1 | 72.8 | 57.0 | | |
| 1-2:2 | 10 l | 30.6 | 105.6 | 19.7 | 49% | 26% |
| 1-2:5 | 10 | 14.9 | 35.8 | 28.3 | | |
| 1-2:3 | 22 l | 98.0 | 88.5 | 75.2 | 54% | 6% |
| 1-2:6 | 22 | 43.7 | 36.5 | 81.5 | | |
| 1-3:1 | 4 l | 144.1 | 105.1 | 93.1 | 3% | 13% |
| 1-3:4 4 | | 137.3 | 83.2 | 112.2 | | |
| 1-3:2 | 16 l | 102.0 | 104.8 | 66.1 | 56% | 10% |
| 1-3:5 | 16 | 45.1 | 40.1 | 76.4 | | |
| 1-3:3 | 23 l | 0.0 | 89.7 | 0.0 | | |
| 1-3:6 | 23 | 1.0 | 35.2 | 2.0 | | |
| 1-4:1 | 5 l | 87.8 | 113.7 | 52.5 | 5% | 11% |
| 1-4:4 | 5 | 82.2 | 90.9 | 61.4 | | |
| 1-4:2 | 20 l | 5.0 | 109.1 | 3.1 | 45% | 14% |
| 1-4:5 | 20 | 2.6 | 46.7 | 3.8 | | |
| 1-4:3 | 18 l | 0.0 | 81.4 | 0.0 | | |
| 1-4:6 | 18 | 0.6 | 38.6 | 1.0 | | |
| | | | 67.9 | | 34% | 15% |

## Claims

1. Method for the identification of an analyte in a biological sample comprising the steps of:
(a) providing a microarray comprising a substrate with predefined regions wherein each binding substance immobilized at a predefined region onto said substrate comprises a predetermined amount of receptor and a predetermined amount of an internal reference,
(b) providing a reporter molecule that binds selectively to said internal reference,
(c) adding said reporter molecule to said biological sample,
(d) contacting said biological sample comprising said reporter molecule with said microarray under conditions that allow binding to take place between said receptor and said analyte, and between said internal reference and said reporter molecule,
(e) determining the signal of said reporter molecule binding to said internal reference,
(f) determining the signal of said analyte binding to said receptor, and,
(g) normalising said signal of step (f) for said signal of step (e), whereby said analyte is identified.

2. The method according to claim 1, wherein said microarray is a flow-through microarray.

3. The method according to any of claims 1 or 2, wherein said substrate is a porous substrate.

4. The method according to any of claims 1 to 3, wherein said substrate is an electrochemically manufactured metal oxide membrane.

5. The method according to any claims 1 to 4, wherein said substrate comprises aluminium oxide.

6. The method according to any of claims 1 to 5, wherein said internal reference comprises polynucleic adds or (poly)peptides or chemical compounds.

7. The method according to any of claims 1 to 6, wherein said each binding substance immobilized onto said substrate comprises at least 1% to at most 99% of said internal reference.

8. The method according to any of claims 1 to 7, wherein said each binding substance immobilized onto said substrate comprises the same predetermined amount of said internal reference.

9. The method according to any of claims 1 to 8, wherein said receptor and said internal reference are separate molecules.

10. Method for the normalization of a microarray comprising the steps of:
(a) immobilizing onto said array a binding substance comprising a receptor and a predetermined amount of an internal reference, and,
(b) determining the signal generated by said internal reference by means of a reporter molecule which selectively binds to said internal reference.

11. The method according to any of claims 1 to 10, wherein said reporter molecule comprises polynucleic adds, (poly)peptides or chemical compounds.

12. The method according to any of claims 1 to 11, wherein said reporter molecule comprises a label.

13. The method according to daim 12, wherein said label is of the enzymatic, fluorescent, phosphorescent or radioactive type.

14. The method according to any of claims 1 to 13 wherein said internal reference comprises nucleic acids.

15. The method according to any of claims 1 to 14, wherein said analyte is labeled.

16. The method according to claim 15, wherein said label is of the enzymatic, fluorescent, phosphorescent or radioactive type.

17. The method according to any of claims 1 to 16, wherein the label of said analyte differs from the label of said internal reference.

18. The method according to claim. 17, wherein the label of said analyte is Texas red, and the label of said internal reference is fluorescein.

19. The method according to claim 17, wherein the label of said internal reference is Texas red, and the label of said analyte is fluorescein.

20. The method according to any of claims 1 to 19 for use in expression profiling assay, genotyping, sequence determination by hybridization, gene quantitation, gene abnormality analysis (MAPH), PCR, NASBA, or TYRAS.

21. Use of a microarray for normalisation of analyte variation, wherein said microarray comprises a substrate with predefined regions, wherein each binding substance immobilized at a predefined region of said substrate comprises a receptor and a predetermined amount of an internal reference, wherein the signal generated by said internal reference is determined by means of a reporter molecule and wherein said reporter molecule selectively binds to said internal reference.

22. Use of a microarray in the method according to any of claims 1 to 20.

23. Microarray for use in the method according to any of claims 1 to 20, wherein said microarray comprises a substrate with predefined regions, wherein each binding substance immobilized at a predefined region of said substrate comprises a receptor and a predetermined amount of an internal reference, wherein the signal generated by said internal reference is determined by means of a reporter molecule and wherein said reporter molecule selectively binds to said internal reference.

24. Device or kit comprising a flow-through based microarray according to claim 23.

25. Use of a device or kit according to daim 24 in expression profiling assay, genotyping, sequence determination by hybridization, gene quantitation, gene abnormality analysis (MAPH), PCR, NASBA, orTYRAS.

26. Use of a reporter molecule for the manufacture of a device or kit according to daim 24.

27. Use of an internal reference for the manufacture of a kit or device according to daim 24.

28. Method for correlating variation in analytes, comprising:
(a) providing at least two analytes, wherein each analyte is identified according to the method according to claim 1,
(b) comparing the values of the analytes of step (g) as defined in claim 1, whereby variation in analytes is correlated.

29. Method of generating a report that correlates analyte variation determined by a method according to any of the previous claims.

## Patentansprüche

1. Verfahren zur Identifizierung eines Analyten in einer biologischen Probe, wobei das Verfahren die Schritte umfasst:
(a) eine Mikro-Anordnung (microarray) zur Verfügung zu stellen, die ein Substrat mit definierten Regionen umfasst, wobei jede bindende Substanz, die an einer definierten Region an dem Substrat immobilisiert ist, eine vorweg bestimmte Menge von Rezeptor und eine vorweg bestimmte Menge eines internen Standards umfasst,
(b) ein Reporter-Molekül zur Verfügung zu stellen, das selektiv an den internen Standard bindet,
(c) das Reporter-Molekül der biologischen Probe zuzusetzen,
(d) die biologische Probe, die das Reporter-Molekül umfasst, mit der Mikro-Anordnung unter Bedingungen in Kontakt zu bringen, die die Ausbildung einer Bindung zwischen Rezeptor und Analyt und zwischen internem Standard und dem Reporter-Molekül ermöglicht,
(e) das Signal des Reporter-Moleküls, das an den internen Standard bindet, zu bestimmen,
(f) das Signal des Analyten, der an den Rezeptor bindet, zu bestimmen, und
(g) das Signal aus Schritt (f) für das Signal von Schritt (e) zu normalisieren, wobei der Analyt identifiziert wird.

2. Das Verfahren nach Anspruch 1, wobei die Mikro-Anordnung eine Durchfluss-Mikro-Anordnung ist.

3. Das Verfahren nach einem der Ansprüche 1 oder 2, wobei das Substrat ein poröses Substrat ist.

4. Das Verfahren nach einem der Ansprüche 1 bis 3, wobei das Substrat eine elektrochemisch hergestellte Metalloxid-Membran ist.

5. Das Verfahren nach einem der Ansprüche 1 bis 4, wobei das Substrat Aluminiumoxid umfasst.

6. Das Verfahren nach einem der Ansprüche 1 bis 5, wobei der interne Standard Polynukleinsäuren oder (Poly)Peptide oder chemische Verbindungen umfasst.

7. Das Verfahren nach einem der Ansprüche 1 bis 6, wobei jede bindende Substanz, die an dem Substrat immobilisiert ist, mindestens 1% bis maximal 99% des internen Standards umfasst.

8. Das Verfahren nach einem der Ansprüche 1 bis 7, wobei jede bindende Substanz, die an dem Substrat immobilisiert ist, dieselbe vorab bestimmte Menge des internen Standards umfasst.

9. Das Verfahren nach einem der Ansprüche 1 bis 8, wobei der Rezeptor und der interne Standard verschiedene Moleküle sind.

10. Verfahren für die Normalisierung einer Mikro-Anordnung, wobei das Verfahren die Schritte umfasst:
(a) eine bindende Substanz, die einen Rezeptor und eine vorweg bestimmte Menge eines internen Standards umfasst, an der Anordnung zu immobilisieren und
(b) das durch den internen Standard mittels eines Reporter-Moleküls, das an den internen Standard selektiv bindet, erzeugte Signal zu bestimmen.

11. Das Verfahren nach einem der Ansprüche 1 bis 10, wobei das Reporter-Molekül Polynukleinsäuren, (Poly)Peptide oder chemische Verbindungen umfasst.

12. Das Verfahren nach einem der Ansprüche 1 bis 11, wobei das Reporter-Molekül eine Markierung umfasst.

13. Das Verfahren nach Anspruch 12, wobei die Markierung eine enzymatische, eine Fluoreszenz-, eine Phosphoreszenz- oder eine Radioaktiv-Markierung ist.

14. Das Verfahren nach einem der Ansprüche 1 bis 13, wobei der interne Standard Nukleinsäuren umfasst.

15. Das Verfahren nach einem der Ansprüche 1 bis 14, wobei der Analyt markiert ist.

16. Das Verfahren nach Anspruch 15, wobei die Markierung eine enzymatische, eine Fluoreszenz-, eine Phosphoreszenz- oder eine Radioaktiv-Markierung ist.

17. Das Verfahren nach einem der Ansprüche 1 bis 16, wobei die Markierung des Analyten und die Markierung des internen Standards unterschiedlich sind.

18. Das Verfahren nach Anspruch 17, wobei die Markierung des Analyten Texas-rot ist, und die Markierung des internen Standards fluorescein ist.

19. Das Verfahren nach Anspruch 17, wobei die Markierung des internen Standards Texas-rot ist, und die Markierung des Analyten fluorescein ist.

20. Das Verfahren nach einem der Ansprüche 1 bis 19 für die Verwendung in einem Assay zur Expressionsdarstellung (expression profiling) in einem Verfahren zur Bestimmung des Genotyps, bei der Sequenz-Bestimmung durch Hybridisierung, für die Gen-Quantifizierung, für die Analyse auf Gen-Abnormalität (MAPH), für PCR, für NASBA oder für TYRAS.

21. Verwendung einer Mikro-Anordnung für die Normalisierung einer Variation von Analyt, wobei die Mikro-Anordnung ein Substrat mit vorweg definierten Regionen umfasst, wobei jede bindende Substanz, die an einer vorweg definierten Region des Substrates immobilisiert ist, einen Rezeptor und eine vorweg bestimmte Menge eines internen Standards umfasst, wobei das Signal, das durch den internen Standard erzeugt wird, mittels eines Reporter-Moleküls bestimmt wird, und wobei das Reporter-Molekül den internen Standard selektiv bindet.

22. Verwendung einer Mikro-Anordnung in dem Verfahren nach einem der Ansprüche 1 bis 20.

23. Mikro-Anordnung für die Verwendung in dem Verfahren nach einem der Ansprüche 1 bis 20, wobei die Mikro-Anordnung ein Substrat mit vorweg definierten Regionen umfasst, wobei jede bindende Substanz, die an einer vorweg definierten Region des Substrates immobilisiert ist, einen Rezeptor und eine vorweg bestimmte Menge eines internen Standards umfasst, wobei das Signal, das durch den internen Standard erzeugt wird, mittels eines Reporter-Moleküls bestimmt wird, und wobei das Reporter-Molekül den internen Standard selektiv bindet.

24. Vorrichtung oder Kit umfassend eine Mikro-Anordnung auf Durchfluss-Basis nach Anspruch 23.

25. Verwendung einer Vorrichtung oder eines Kits nach Anspruch 24 in einem Assay zur Expressionsdarstellung in einem Verfahren zur Bestimmung des Genotyps, bei der Sequenz-Bestimmung durch Hybridisierung, für die Gen-Quantifizierung, für die Analyse auf Gen-Abnormalität (MAPH), für PCR, für NASBA oder für TYRAS.

26. Verwendung eines Reporter-Moleküls für die Herstellung einer Vorrichtung oder eines Kits nach Anspruch 24.

27. Verwendung eines internen Standards für die Herstellung eines Kits oder einer Vorrichtung nach Anspruch 24.

28. Verfahren um die Variation in Analyten zu korrelieren, wobei das Verfahren die Schritte umfasst:
(a) wenigstens zwei Analyte zur Verfügung zu stellen, wobei jeder Analyt nach dem Verfahren nach Anspruch 1 identifiziert wird,
(b) die Werte der Analyten in gemäß Anspruch 1 definiertem Schritt (g) zu vergleichen, wobei die Variation in den Analyten korreliert wird.

29. Verfahren zur Erstellung eines Berichts, der die Variation der Analyte korreliert, wie sie mittels eines Verfahrens nach einem der vorhergehenden Ansprüche bestimmt wurde.

## Revendications

1. Procédé d'identification d'un analyte dans un échantillon biologique, comprenant les étapes consistant à :
(a) fournir un micro-réseau comprenant un support avec des régions prédéfinies, dans lequel chaque substance de liaison immobilisée dans une région prédéfinie sur ledit support comprend une quantité prédéterminée de récepteur et une quantité prédéterminée d'une référence interne,
(b) fournir une molécule rapporteur qui se lie sélectivement à ladite référence interne,
(c) ajouter ladite molécule rapporteur audit échantillon biologique,
(d) mettre en contact ledit échantillon biologique comprenant ladite molécule rapporteur avec ledit micro-réseau dans des conditions qui permettent à la liaison d'avoir lieu entre ledit récepteur et ledit analyte et entre ladite référence interne et ladite molécule rapporteur,
(e) déterminer le signal de ladite molécule rapporteur qui se lie à ladite référence interne,
(f) déterminer le signal dudit analyte qui se lie audit récepteur, et
(g) normaliser ledit signal de l'étape (f) pour ledit signal de l'étape (e), de sorte que ledit analyte est identifié.

2. Procédé selon la revendication 1, dans lequel ledit micro-réseau est un micro-réseau de circulation d'un écoulement.

3. Procédé selon l'une quelconque des revendications 1 ou 2, dans lequel ledit support est un support poreux.

4. Procédé selon l'une quelconque des revendications 1 à 3, dans lequel ledit support est une membrane d'oxyde métallique fabriquée de façon électrochimique.

5. Procédé selon l'une quelconque des revendications 1 à 4, dans lequel ledit support comprend de l'oxyde d'aluminium.

6. Procédé selon l'une quelconque des revendications 1 à 5, dans lequel ladite référence interne comprend des acides polynucléiques ou des (poly)peptides ou des composés chimiques.

7. Procédé selon l'une quelconque des revendications 1 à 6, dans lequel chaque substance de liaison immobilisée sur ledit support comprend au minimum 1 % à 99 % au maximum de ladite référence interne.

8. Procédé selon l'une quelconque des revendications 1 à 7; dans lequel chaque substance de liaison immobilisée sur ledit support comprend la même quantité prédéterminée de ladite référence interne.

9. Procédé selon l'une quelconque des revendications 1 à 8, dans lequel ledit récepteur et ladite référence interne sont des molécules séparées.

10. Procédé de normalisation d'un micro-réseau comprenant les étapes consistant à :
(a) immobiliser sur ledit réseau une substance de liaison comprenant un récepteur et une quantité prédéterminée d'une référence interne, et
(b) déterminer le signal généré par ladite référence interne au moyen d'une molécule rapporteur qui se lie sélectivement à ladite référence interne.

11. Procédé selon l'une quelconque des revendications 1 à 10, dans lequel ladite molécule rapporteur comprend des acides polynucléiques, des (poly)peptides ou des composés chimiques.

12. Procédé selon l'une quelconque des revendications 1 à 11, dans lequel ladite molécule rapporteur comprend un marqueur.

13. Procédé selon la revendication 12, dans lequel ledit marqueur est du type enzymatique, fluorescent, phosphorescent ou radioactif.

14. Procédé selon l'une quelconque des revendications 1 à 13, dans lequel ladite référence interne comprend des acides nucléiques.

15. Procédé selon l'une quelconque des revendications 1 à 14, dans lequel ledit analyte est marqué.

16. Procédé selon la revendication 15, dans lequel ledit marqueur est du type enzymatique, fluorescent, phosphorescent ou radioactif.

17. Procédé selon l'une quelconque des revendications 1 à 16, dans lequel le marqueur dudit analyte diffère du marqueur de ladite référence interne.

18. Procédé selon la revendication 17, dans lequel le marqueur dudit analyte est rouge Texas et le marqueur de ladite référence interne est de la fluorescéine.

19. Procédé selon la revendication 17, dans lequel le marqueur de ladite référence interne est rouge Texas et le marqueur dudit analyte est de la fluorescéine.

20. Procédé selon l'une quelconque des revendications 1 à 19, pour utilisation dans l'analyse de profilage d'expression, le génotypage, la détermination de séquences par l'hybridation, le dosage des gènes, l'analyse des anomalies des gènes (MAPH), la réaction de polymérisation en chaîne (PCR), la réaction d'amplification de l'acide nucléique basée sur la séquence (NASBA), ou la réaction « TYRAS ».

21. Utilisation d'un micro-réseau pour normalisation de la variation de l'analyte, dans laquelle ledit micro-réseau comprend un support avec des régions prédéfinies, dans lequel chaque substance de liaison immobilisée dans une région prédéfinie dudit support comprend un récepteur et une quantité prédéterminée d'une référence interne, le signal généré par ladite référence interne étant déterminé au moyen d'une molécule rapporteur et ladite molécule rapporteur se liant sélectivement à ladite référence interne.

22. Utilisation d'un micro-réseau dans le procédé selon l'une quelconque des revendications 1 à 20.

23. Micro-réseau pour utilisation dans le procédé selon l'une quelconque des revendications 1 à 20, dans lequel ledit micro-réseau comprend un support avec des régions prédéfinies, dans lequel chaque substance de liaison immobilisée dans une région prédéfinie dudit support comprend un récepteur et une quantité prédéterminée d'une référence interne, le signal généré par ladite référence interne étant déterminé au moyen d'une molécule rapporteur et ladite molécule rapporteur se liant sélectivement à ladite référence interne.

24. Dispositif ou kit comprenant un micro-réseau basé sur la circulation d'un écoulement selon la revendication 23.

25. Utilisation d'un dispositif ou kit selon la revendication 24 dans l'analyse de profilage d'expression, le génotypage, la détermination de séquences par l'hybridation, le dosage des gènes, l'analyse des anomalies des gènes (MAPH), la réaction en chaîne par polymérase, la réaction d'amplification de l'acide nucléique basée sur la séquence (NASBA), ou la réaction « TYRAS ».

26. Utilisation d'une molécule rapporteur pour la fabrication d'un dispositif ou kit selon la revendication 24.

27. Utilisation d'une référence interne pour la fabrication d'un kit ou dispositif selon la revendication 24.

28. Procédé pour corréler la variation dans des analytes, consistant à :
(a) fournir au moins deux analytes, chaque analyte étant identifié selon le procédé selon la revendication 1,
(b) comparer les valeurs des analytes de l'étape (g) telles que définie dans la revendication 1, de sorte que la variation dans les analytes est corrélée.

29. Procédé de génération d'un rapport qui corrèle la variation dans les analytes, déterminée par un procédé selon l'une quelconque des revendications précédentes.


### SEQUENCE LISTING

<110> PamGene B.V.
<120> Normalisation of microarray data based on hybridisation with an internal reerence
<130> PAM-005-PCT
<150> EP 01870295.1
   <151> 2001-12-21
<150> US 60/383,666
   <151> 2002-05-28
<160> 18
<170> Patent In version 3.1
<210> 1
   <211> 21
   <212> DNA
   <213> Artificial Sequence
<220>
   <223> Oligonucleotide
<400> 1
   tgcaaagtat catccctcca g 21
<210> 2
   <211> 21
   <212> DNA
   <213> Artificial Sequence
<220>
   <223> Oligonucleotide
<400> 2
   ctggagggat gatactttgc a 21
<210> 3
   <211> 21
   <212> DNA
   <213> Artificial Sequence
<220>
   <223> Oligonucleotide
<400> 3
   ctggagggat gatactttgc a 21
<210> 4
   <211> 21
   <212> DNA
   <213> Artificial Sequence
<220>
   <223> Oligonucleotide
<400> 4
   ctggagggat gatactttgc a 21
<210> 5
   <211> 21
   <212> DNA
   <213> Artificial Sequence
<220>
   <223> Oligonucleotide
<400> 5
   tccttttcca gttctgtaca a 21
<210> 6
   <211> 22
   <212> DNA
   <213> Artificial Sequence
<220>
   <223> Oligonucleotide
<400> 6
   catgtatcga ggataaatga ag 22
<210> 7
   <211> 21
   <212> DNA
   <213> Artificial Sequence
<220>
   <223> Oligonucleotide
<400> 7
   ttgtacagag atggaaaagg a 21
<210> 8
   <211> 21
   <212> DNA
   <213> Artificial Sequence
<220>
   <223> Oligonucleotide
<400> 8
   ttgtacagaa ctggaaaagg a 21
<210> 9
   <211> 21
   <212> DNA
   <213> Artificial Sequence
<220>
   <223> Oligonucleotide
<400> 9
   ttgtgcagaa atggaaaagg a 21
<210> 10
   <211> 21
   <212> DNA
   <213> Artificial Sequence
<220>
   <223> Oligonucleotide
<400> 10
   ttgtacagaa atggaaaaag a 21
<210> 11
   <211> 21
   <212> DNA
   <213> Artificial Sequence
<220>
   <223> Oligonucleotide
<400> 11
   ttgcacagaa atggaaaagg a 21
<210> 12
   <211> 21
   <212> DNA
   <213> Artificial Sequence
<220>
   <223> Oligonucleotide
<400> 12
   ttgtacagaa ctggagaagg a 21
<210> 13
   <211> 21
   <212> DNA
   <213> Artificial Sequence
<220>
   <223> Oligonucleotide
<400> 13
   ttgtaaagag atggaacagg a 21
<210> 14
   <211> 21
   <212> DNA
   <213> Artificial Sequence
<220>
   <223> Oligonucleotide
<400> 14
   ttgtgcagat atggaaaagg a 21
<210> 15
   <211> 21
   <212> DNA
   <213> Artificial Sequence
<220>
   <223> Oligonucleotide
<400> 15
   ttgtgcattt atggaggagg a 21
<210> 16
   <211> 21
   <212> DNA
   <213> Artificial Sequence
<220>
   <223> Oligonucleotide
<400> 16
   ttgtacagaa ttggaaaagg a 21
<210> 17
   <211> 21
   <212> DNA
   <213> Artificial Sequence
<220>
   <223> Oligonucleotide
<400> 17
   ttgtttagaa atggaaaagg a 21
<210> 18
   <211> 21
   <212> DNA
   <213> Artificial Sequence
<220>
   <223> Oligonucleotide
<400> 18
   tccttttcca gttctgtaca a 21